# EUROPEAN PATENT APPLICATION

(11) **EP 3 973 997 A1**
(43) Date of publication of application: **30.03.2022**
(21) Application number: 21201467.4
(22) Date of filing: 23.04.2018
(51) Int. Cl.: A61K 48/00

(54) **MEMBRANE ACTIVE MOLECULES**

(30) Priority: 24.04.2017 US 201762489352 P; 24.05.2017 US 201762510705 P
(62) Divisional of application: 18791339.7
(71) Applicant: Branequest, Inc., Pasadena, CA 91103-3539 (US)
(72) Inventor: SONNER, James M., Pasadena, 91103-3539 (US); KRSTENANSKY, John L., Redlands, 92373 (US)
(74) Representative: J A Kemp LLP

(57) **Abstract**

A method of inducing anesthesia, sedation, and a method for treating disorders including central nervous system disorder, peripheral nervous system disorder, depression ischemia, and treatment with an anticonvulsant by administering an effective amount of a compound to a subject in need thereof.

## Description

### CROSS-REFERENCE

This application claims priority to U.S. Provisional Patent Application Serial No. 62/489,352, filed April 24, 2017, and U.S. Provisional Patent Application Serial No. 62/510,705, filed May 24, 2017, each of which is entirely incorporated herein by reference.

### BACKGROUND

In surgical procedures, anesthetics may be used to reduce the pain a subject may feel during surgery. However, common anesthetics may incur a number of undesired side effects, some lasting for days after the surgery. There exists an unmet need to develop new methods of inducing anesthesia with a reduced number and level of side effects.

### SUMMARY

Disclosed herein are new methods for inducing anesthesia and for treating disorders comprising administering a compound to a subject in need thereof.

One aspect of the current disclosure is a method comprising administering to a subject in need thereof a therapeutically-effective amount of a compound of Formula (I): 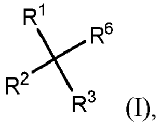 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
R⁸ is independently selected at each occurrence from hydrogen and C₁₋₂₀ alkyl; and
wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof.

In some embodiments, the compound is represented by Formula (I-A): 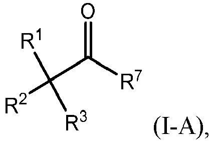 or a salt thereof.

One aspect of the current disclosure is a method comprising administering to a subject in need thereof a therapeutically-effective amount of a compound or a salt thereof listed in Table 1 or Table 2, wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof.

Another aspect of the current disclosure is a pharmaceutical composition comprising a compound of Formula (I): 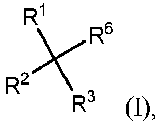 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
R⁸ is independently selected at each occurrence from hydrogen and C₁₋₂₀ alkyl; and a pharmaceutically-acceptable excipient.

In some embodiments, the compound is represented by Formula (I-A): 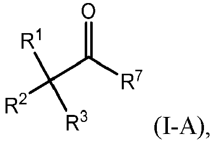 or a salt thereof.

Another aspect of the current disclosure is a pharmaceutical composition comprising a therapeutically-effective amount of a compound or a salt thereof listed in Table 1 or Table 2, and a pharmaceutically-acceptable excipient.

Another aspect of the current disclosure is a compound of Formula (I): 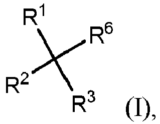 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S; and
R⁸ is independently selected at each occurrence from hydrogen and C₁₋₂₀ alkyl.

Another aspect of the current disclosure is a method of combining a compound of Formula (I): 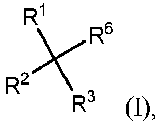 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
R⁸ is independently selected at each occurrence from hydrogen and C₁₋₂₀ alkyl, and a pharmaceutically-acceptable excipient.

In an aspect, the present disclosure provides a method comprising administering to a subject in need thereof a therapeutically-effective amount of a compound of Formula (I): 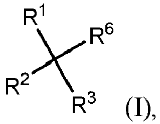 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
R⁸ is independently selected at each occurrence from hydrogen and C₁₋₂₀ alkyl; and
wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof.

In some embodiments, the compound is represented by Formula (I-A): 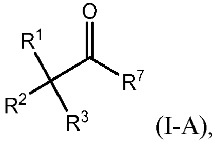 or a salt thereof.

In an aspect, the present disclosure provides a method comprising administering to a subject in need thereof a therapeutically-effective amount of a compound or a salt thereof listed in Table 1 or Table 2, wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof. In some embodiments, the compound or a salt thereof is a racemic mixture. In some embodiments, the compound or a salt thereof has an enantiomeric excess of greater than 80%. In some embodiments, the compound or a salt thereof has a diastereomeric excess of greater than 80%. In some embodiments, the compound or a salt thereof has an alkene group, and wherein said alkene group has a cis geometry. In some embodiments, the compound or a salt thereof has an alkene group, and wherein said alkene group has a trans geometry. In some embodiments, the compound or a salt thereof is administered in a formulation. In some embodiments, the formulation further comprises a pharmaceutically-acceptable excipient. In some embodiments, the formulation is administered orally. In some embodiments, the formulation is administered topically. In some embodiments, the formulation is administered by inhalation. In some embodiments, the formulation is administered intravenously. In some embodiments, the formulation is administered intramuscularly. In some embodiments, the formulation is administered by spinal delivery. In some embodiments, the formulation is administered by epidural delivery. In some embodiments, the administering of a compound or a salt thereof alters distribution of lipids in a cell membrane. In some embodiments, the administering of a compound or a salt thereof alters cell membrane thickness. In some embodiments, the compound or a salt thereof induces lower than about 50% of sides effects, comprising nausea, vomiting, sore throat, confusion, hypothermia, respiratory depression, low blood pressure, or any combination thereof, when compared to an anesthetics selected from the group consisting of desflurane, procaine, lidocaine, cocaine, amobarbital, sevoflurane, isoflurane, etomidate, ketamine, ropivicaine, bupivicaine, propofol, and alfentanil. In some embodiments, the compound or salt thereof demonstrates a lower level of toxicity to a subject when compared to anesthetics selected from the group consisting of desflurane, procaine, lidocaine, cocaine, amobarbital, alfentanil, etomidate, propofol, ketamine, isoflurane, sevoflurane, ropivicaine, and bupivicaine. In some embodiments, the method is a method of inducing sedation or sedating said subject. In some embodiments, the method induces minimal sedation. In some embodiments, the method is a method of treating a central nervous system disorder. In some embodiments, the central nervous system disorder is one selected from the group consisting of schizophrenia, bipolar disorder, autism, Alzheimer's disease, Parkinson's disease, attention deficit-hyperactivity disorder, and sleep disorders. In some embodiments, the method is a method of treating a peripheral nervous system disorder. In some embodiments, the peripheral nervous system disorder is one selected from the group consisting of traumatic nerve damage, diabetic neuropathy, chemotherapy induced neuropathy, spinal muscular atrophy, restless leg syndrome, and motor neuron disease. In some embodiments, the method is a method of treating a convulsing disorder. In some embodiments, the convulsing disorder is epilepsy. In some embodiments, the method is used to In some embodiments, the method reduces the frequency and/or severity of a twitching in a subject. In some embodiments, the method reduces the frequency and/or severity of a seizure in a subject. In some embodiments, the method is a method of treating a psychiatric disorder. In some embodiments, the psychiatric disorder is one selected from the group consisting of attention deficit hyperactivity disorder, alcohol abuse, depression, panic disorder, posttraumatic stress disorder, and schizophrenia. In some embodiments, the method is a method of treating an ischemia. In some embodiments, the ischemia is one selected from the group consisting of myocardial ischemia, cerebral ischemia, and limb ischemia. In some embodiments, the method is a method of treating pain. In some embodiments, the pain is acute pain. In some embodiments, the pain is chronic pain. In some embodiments, the pain is associated with a disease. In some embodiments, the method is a method of treating spasticity. In some embodiments, the spasticity level in said subject decreases after a treatment with said compound or a salt thereof. In some embodiments, the method further comprises administering a pharmaceutically-acceptable excipient. In some embodiments, the compound or a salt thereof is administered in an amount from about 0.001 mg to about 10,000 mg per kg body weight. In some embodiments, the compound or a salt thereof is administered in an amount from about 0.1 mg to about 1,000 mg per kg body weight. In some embodiments, the compound or a salt thereof is administered at least 1 time per week. In some embodiments, the compound or a salt thereof is administered at least 1 time per day. In some embodiments, the administering occurs for a length of time from about 1 second to about 100 minutes. In some embodiments, the compound or salt thereof is selected from the group consisting of: (S)-3-hydroxybutanoic acid, (R)-3-hydroxybutanoic acid, 3,3-dimethylbutanoic acid, 2-benzamido-2-hydroxyacetic acid, butyric acid, 2-ethylmalonic acid, glutamine, and a salt of any one thereof. In some embodiments, the compound or salt thereof is selected from the group consisting of: butyric acid, 3-hydroxybutyric acid, ethylmalonic acid, diethyl ethyl malonate, ethyl (S)-(+)-mandelate, ethyl isovaleric acid, ethyl butyrate, diethyl ethylphenylmalonate, and a salt of any one thereof. In some embodiments, the compound or salt thereof is selected from the group consisting of: butyric acid, 3-hydroxybutyric acid, ethylmalonic acid, diethyl ethyl malonate, and a salt of any one thereof. In some embodiments, the compound or salt thereof is butyric acid. In some embodiments, the compound or salt thereof is 3-hydroxybutyric acid.

In some aspects, the present disclosure provides a pharmaceutical composition comprising a compound of Formula (I): 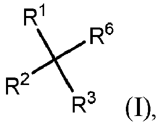 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
R⁸ is independently selected at each occurrence from hydrogen and C₁₋₂₀ alkyl; and a pharmaceutically-acceptable excipient.

In some embodiments, the compound is represented by Formula (I-A): 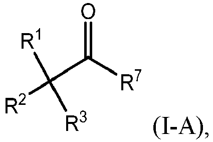 or a salt thereof.
In some aspects, the present disclosure provides a pharmaceutical composition comprising a therapeutically-effective amount of a compound or a salt thereof listed in Table 1 or Table 2, and a pharmaceutically-acceptable excipient. In some embodiments, the compound or salt thereof is butyric acid. In some embodiments, the compound or salt thereof is 3-hydroxybutyric acid. In some embodiments, the pharmaceutically-acceptable excipient is selected from the group consisting of water, alcohol, glycerol, chitosan, alginate, chondroitin, Vitamin E, mineral oil, and dimethyl sulfoxide, lactose, dextrose, sucrose, sorbitol, mannitol, starches, gum acacia, calcium phosphate, alginates, tragacanth, gelatin, calcium silicate, microcrystalline cellulose, PEG, polyvinylpyrrolidone, cellulose, sterile saline, syrup, methyl cellulose, and any combination thereof. In some embodiments, the compound or a salt thereof is a racemic mixture. In some embodiments, the compound or a salt thereof has an enantiomeric excess of greater than 80%. In some embodiments, the compound or a salt thereof has a diastereomeric excess of greater than 80%. In some embodiments, the compound or a salt thereof has an alkene group, and wherein said alkene group has a cis geometry. In some embodiments, the compound or a salt thereof has an alkene group, and wherein said alkene group has a trans geometry.

In some aspects, the present disclosure provides a compound of Formula (I): 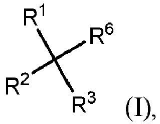 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S; and
R⁸ is independently selected at each occurrence from hydrogen and C₁₋₂₀ alkyl.

In an aspect, the compound is represented by Formula (I-A): 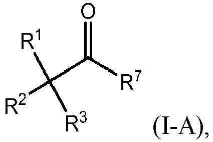 or a salt thereof.

In an aspect, the present disclosure provides a compound or a salt thereof listed in Table 1 or Table 2. In some embodiments, the compound or a salt thereof is a racemic mixture. In some embodiments, the compound or a salt thereof has an enantiomeric excess of greater than 80%. In some embodiments, the compound or a salt thereof has a diastereomeric excess of greater than 80%. In some embodiments, the compound or a salt thereof has an alkene group, and wherein said alkene group has a cis geometry. In some embodiments, the compound or a salt thereof has an alkene group, and wherein said alkene group has a trans geometry.
In an aspect, the present disclosure provides a method of combining a compound of Formula (I): 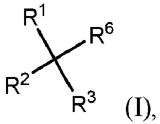 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S,
R⁸ is independently selected at each occurrence from hydrogen and C₁₋₂₀ alkyl, and a pharmaceutically-acceptable excipient.

In some embodiments, the excipient is selected from the group consisting of water, alcohol, glycerol, chitosan, alginate, chondroitin, Vitamin E, mineral oil, and dimethyl sulfoxide, lactose, dextrose, sucrose, sorbitol, mannitol, starches, gum acacia, calcium phosphate, alginates, tragacanth, gelatin, calcium silicate, microcrystalline cellulose, PEG, polyvinylpyrrolidone, cellulose, sterile saline, syrup, methyl cellulose, and any combination thereof. In some embodiments, the method further comprises employing the compound of Formula I. In some embodiments, the method further comprises employing the salt of the compound of Formula I.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

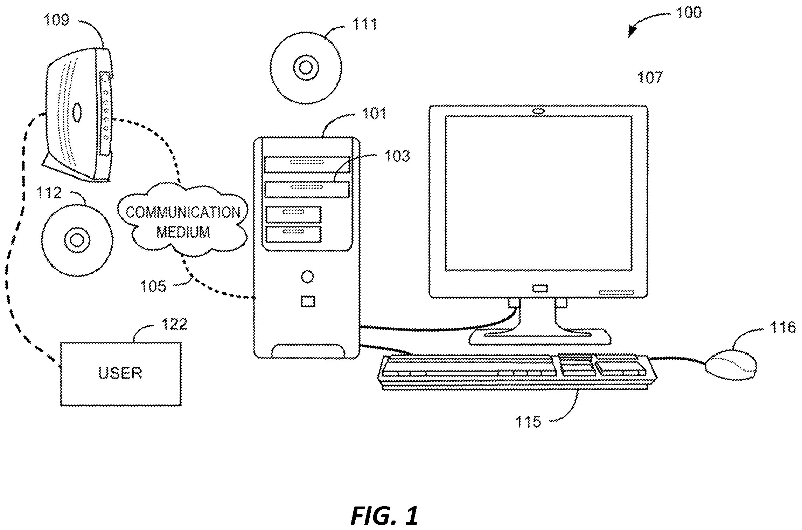

Figure 1 illustrates an example of a computer system that can be used in connection with example embodiments of the present disclosure.

### DETAILED DESCRIPTION

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

### Pharmaceutical Compositions

The term "salt" refers to salts derived from a variety of organic and inorganic counter ions well known in the art. Acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p toluenesulfonic acid, salicylic acid, and the like. Base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the base addition salt is chosen from ammonium, potassium, sodium, calcium, and magnesium salts.

The term "pharmaceutically-acceptable carrier" or "pharmaceutically-acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions of the disclosure is contemplated. Supplementary active ingredients can also be incorporated into the compositions.

The term "pharmaceutically-acceptable excipient" is intended to include vehicles and carriers capable of being coadministered with a compound to facilitate the performance of its intended function. The use of such media for pharmaceutically active substances is well known in the art. Examples of such vehicles and carriers include solutions, solvents, dispersion media, delay agents, emulsions and the like. Any other conventional carrier suitable for use with the multi-binding compounds also falls within the scope of the present disclosure.

The term "subject" includes, but is not limited to, humans of any age group, e.g., a pediatric subject (e.g., infant, child or adolescent) or adult subject (e.g., young adult, middle-aged adult or senior adult)) and/or other primates (e.g., cynomolgus monkeys or rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. The methods described herein can be useful in both human therapeutics and veterinary applications. In some embodiments, the patient is a mammal, and in some embodiments, the patient is human. In some embodiments, a compound of the current disclosure is administered to a subject in need thereof.

The term "therapeutically effective amount" refers to that amount of compound that is sufficient to effect treatment, as defined above, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

Compounds of the present disclosure also include crystalline and amorphous forms of those compounds, pharmaceutically acceptable salts, and active metabolites of these compounds having the same type of activity, including, for example, polymorphs, pseudopolymorphs, solvates, hydrates, unsolvated polymorphs (including anhydrates), conformational polymorphs, and amorphous forms of the compounds, as well as mixtures thereof.

The compounds described herein may exhibit their natural isotopic abundance, or one or more of the atoms may be artificially enriched in a particular isotope having the same atomic number, but an atomic mass or mass number different from the atomic mass or mass number predominantly found in nature. All isotopic variations of the compounds of the present disclosure, whether radioactive or not, are encompassed within the scope of the present disclosure. For example, hydrogen has three naturally occurring isotopes, denoted ¹H (protium), ²H (deuterium), and ³H (tritium). Protium is the most abundant isotope of hydrogen in nature. Enriching for deuterium may afford certain therapeutic advantages, such as increased *in vivo* half-life and/or exposure, or may provide a compound useful for investigating *in vivo* routes of drug elimination and metabolism. Isotopically-enriched compounds may be prepared by conventional techniques well known to those skilled in the art.

In some embodiments, a compound of the disclosure, or its pharmaceutically acceptable salt, comprises 1, 2, 3, 4, 5, or more deuterium atoms. In some embodiments, a hydrogen of a compound of the disclosure, or its pharmaceutically acceptable salt, can be replaced with a deuterium atom.

A compound of the disclosure, or its pharmaceutically acceptable salt, may contain one or more asymmetric centers and may thus give rise to enantiomers, diastereomers, and other stereoisomeric forms that are defined, in terms of absolute stereochemistry, as (R)- or (*S*)- or, as (D)- or (L)- for amino acids. The present disclosure is meant to include all such possible isomers, as well as their racemic and optically pure forms. A "stereoisomer" refers to a compound made up of the same atoms bonded by the same bonds but having different three-dimensional structures, which are not interchangeable. The present disclosure contemplates various stereoisomers and mixtures thereof and includes "enantiomers", which refers to two stereoisomers whose molecules are nonsuperimposeable mirror images of one another. Optically active (+) and (-), (*R*)- and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When a compound or a salt thereof described herein contain olefinic double bonds or other centers of geometric asymmetry, and unless specified otherwise, it is intended that a compound or a salt thereof includes both E and Z geometric isomers.

In some embodiments, a compound or a salt thereof may be dosed in their enantiomerically pure form. In some examples, the compound has an enantiomeric excess greater than about 50%, 60%, 70%, 80%, 90%, 95%, 96%, 97%, 98%, or 99%. A compound or a salt thereof may be dosed in their diasteriomerically pure form. In some examples, the compound has a diasteriomeric excess greater than about 50%, 60%, 70%, 80%, 90%, 95%, 96%, 97%, 98%, or 99%.

Stereocenters may be defined using the Cahn-Ingold-Prelog priority rules. A compound or a salt thereof may have a stereocenter in the *R*-configuration. A compound or a salt thereof may have a stereocenter in the *S*-configuration.

In some cases, a compound or a salt thereof may exhibit polymorphism. It is to be understood that the present disclosure encompasses any racemic, optically-active, polymorphic, or stereoisomeric form, or mixtures thereof, of a compound of the disclosure, which possesses the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase).

In one aspect, the current disclosure provides a method comprising administering to a subject in need thereof a therapeutically-effective amount of a compound of Formula (I): 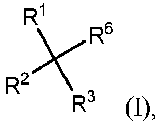 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl;
R¹ and R² together can form a C₃₋₁₀ carbocycle, C₅₋₁₀ aryl, 3- to 10-membered heterocycle, 3- to 10-membered heteroaryl, an oxo, or thio;
X is O or S;
Y is O or S;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, and C₁₋₂₀ alkyl;
R⁵ is independently selected at each occurrence from hydrogen, and C₁₋₂₀ alkyl;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, OR⁵, and NHR⁵; and
wherein the method is selected from inducing sedation, sedating, treating a central nervous system disorder, treating a peripheral nervous system disorder, treating a convulsing disorder, treating a psychiatric disorder, treating ischemia, treating pain, treating spasticity, and treating itching.

In some embodiments, a compound of the current disclosure is represented by Formula (I-A): 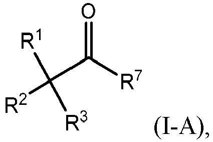 or a salt thereof.

In some embodiments, a compound of the current disclosure is represented by Formula (II): R⁵¹-C(R⁵²)(R^{52'})-C(R⁵³)(R^{53'})-C(R⁵⁴)(R^{54'})(R^{54"}), or a salt thereof, wherein:
R⁵¹ is 5-tetrazolyl or 5-oxo-1,2,4-oxadiazol-3-yl;
R⁵² and R^{52'} are independently selected at each occurrence from hydrogen, amine, or -COOH; or R⁵² and R^{52'} together form an oxo;
R⁵³ is hydrogen, -OH, alkyl, or -COOH;
R^{53'} is hydrogen or methyl; or -H; R⁵³ and R^{53'} together form an oxo;
R⁵⁴ is hydrogen; and
R⁵⁴, R^{54"} , and R^{54"} are independently selected at each occurrence from is selected from hydrogen, -CONH₂, -COCH₃, - COOH, -CH₂COOH; - CH₂CH₂NH₂, or - CH₂NHN(NH)NH₂.

In some embodiments, R⁵¹ is -COOR^{e}, wherein R^{e} is -(CH₂)ₙCH₃, where n is 0-3.

In some embodiments, R⁵¹ is -CONHR^{a}, where R^{a} is hydrogen or alkyl.

In some embodiments, R⁵¹ is -CHO or -CH₂OH.

In some embodiments, R⁵¹ is acyl-OCH₂-.

In some embodiments, R⁵¹ is -COOR^{f}, wherein R^{f} is -(CH₂)ₙCH₃, wherein n is 7-21.

In some embodiments, R⁵¹ is 3-acyloxybutyric acid, where the acyl group is a fatty acid (C8-21).

In some embodiments, a hydrogen of a compound of the current disclosure may be replaced with a deuterium or a halogen, such as, for example, a fluorine.

In some embodiments, a method comprising administering to a subject in need thereof a therapeutically-effective amount of a compound or a salt thereof is listed in Table 1 or Table 2, wherein the method is selected from inducing sedation, sedating, treating a central nervous system disorder, treating a peripheral nervous system disorder, treating a convulsing disorder, treating a psychiatric disorder, treating ischemia, treating pain, treating spasticity, and treating itching.

In some embodiments, the compound or a salt thereof is a racemic mixture. In some embodiments, the compound or a salt thereof has an enantiomeric excess of greater than 80%. In some embodiments, the compound or a salt thereof has a diastereomeric excess of greater than 80%. In some embodiments, the compound or a salt thereof has an alkene group, and wherein said alkene group has a cis geometry. In some embodiments, the compound or a salt thereof has an alkene group, and wherein said alkene group has a trans geometry.

In some embodiments, the compound or a salt thereof is administered in a formulation. In some embodiments, the formulation further comprises an excipient. In some embodiments, the formulation is administered orally. In some embodiments, the formulation is administered topically. In some embodiments, the formulation is administered by inhalation. In some embodiments, the formulation is administered intravenously. In some embodiments, the formulation is administered intramuscularly. In some embodiments, the formulation is administered by spinal delivery. In some embodiments, the formulation is administered by epidural delivery. In some embodiments, the said administering of a compound of Formula (I), or a salt thereof, alters distribution of lipids in a cell membrane. In some embodiments, the administering of a compound of Formula (I) alters cell membrane thickness.

In some embodiments, the compound or a salt thereof of Formula (I) induces lower than about 50% of sides effects, comprising nausea, vomiting, sore throat, confusion side effects, hypothermia, changes in blood pressure, respiratory depression, or any combination thereof, when compared to an anesthetics selected from the group consisting of desflurane, procaine, lidocaine, cocaine, amobarbital, sevoflurane, isoflurane, propofol, etomidate, ketamine and alfentanil.

In some embodiments, the compound or salt thereof of Formula (I) demonstrates a lower level of toxicity to a subject when compared to anesthetics selected from the group consisting of desflurane, procaine, lidocaine, cocaine, amobarbital, sevoflurane, isoflurane, etomidate, ketamine, ropivicaine, bupivicaine, propofol, and alfentanil.

In some embodiments, the method is a method of inducing sedation or sedating said subject. In some embodiments, the method induces minimal sedation. In some embodiments, the method is a method of treating a central nervous system disorder. In some embodiments, the central nervous system disorder is one selected from the group consisting of schizophrenia, bipolar disorder, autism, Alzheimer's disease, Parkinson's disease, attention deficit-hyperactivity disorder, and sleep disorders.

In some embodiments, the method is a method of treating a peripheral nervous system disorder. In some embodiments, the peripheral nervous system disorder is one selected from traumatic nerve damage, diabetic neuropathy, chemotherapy induced neuropathy, spinal muscular atrophy, and motor neuron disease.In some embodiments, the method is a method of treating a convulsing disorder. In some embodiments, the method is used to control and/or prevent seizures or to stop an ongoing series of seizures. In some embodiments, the convulsing disorder is epilepsy. In some embodiments, the convulsing disorder is Dravet syndrome. In some embodiments, the convulsing disorder is Nav 1.1 mutation of genetic epilepsy.

In some embodiments, the method is a method of treating a psychiatric disorder.

In some embodiments, the psychiatric disorder is one selected from attention deficit hyperactivity disorder, alcohol abuse, depression, panic disorder, posttraumatic stress disorder, and schizophrenia. In some embodiments, the method is a method of treating an ischemia. In some embodiments, the ischemia is one selected from myocardial ischemia, cerebral ischemia, and limb ischemia. In some embodiments, the method is a method of treating pain. In some embodiments, the pain is acute pain.In some embodiments, the pain is chronic pain.In some embodiments, the pain is associated with a disease.In some embodiments, the method is a method of treating spasticity. In some embodiments, the spasticity level in said subject decreases after a treatment with said compound.

In one aspect of the current disclosure, a pharmaceutical composition comprises a therapeutically-effective amount of a compound of Formula (I): 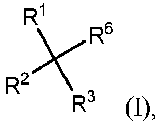 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl;
R¹ and R² together can form a C₃₋₁₀ carbocycle, C₅₋₁₀ aryl, 3- to 10-membered heterocycle, 3- to 10-membered heteroaryl, an oxo, or thio;
X is O or S;
Y is O or S;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, and C₁₋₂₀ alkyl;
R⁵ is independently selected at each occurrence from hydrogen, and C₁₋₂₀ alkyl;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, OR⁵, and NHR⁵; and
a pharmaceutically-acceptable excipient.

In some embodiments, the pharmaceutical composition comprises a compound represented by Formula (I-A): 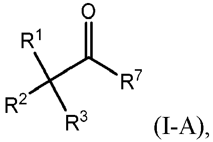 or a salt thereof, and a pharmaceutically-acceptable excipient.

In some embodiments, the pharmaceutical composition comprises a therapeutically-effective amount of a compound or a salt thereof listed in Table 1 or Table 2, and a pharmaceutically-acceptable excipient.

In some embodiments, the pharmaceutical composition comprises a compound, wherein the compound or a salt thereof is a racemic mixture. In some embodiments, the pharmaceutical composition comprises a compound, wherein the compound or a salt thereof has an enantiomeric excess of greater than 80%. In some embodiments, the pharmaceutical composition comprises a compound, wherein the compound or a salt thereof has a diastereomeric excess of greater than 80%. In some embodiments, the pharmaceutical composition comprises a compound, wherein the compound or a salt thereof has an alkene group, and wherein said alkene group has a cis geometry. In some embodiments, the pharmaceutical composition comprises a compound, wherein the compound or a salt thereof has an alkene group, and wherein said alkene group has a trans geometry.

In some embodiments, a pharmaceutical composition disclosed herein comprises a therapeutically-effective amount of a compound or a salt thereof listed in Table 1 or Table 2.

**Table 1.**

| | |
|---|---|
| (S)-3-Hydroxybutanoic acid | 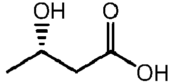 |
| (R)-3-Hydroxybutanoic acid | 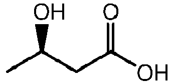 |
| 3,3-Dimethylbutanoic acid | 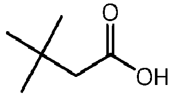 |
| 2-Benzamido-2-hydroxyacetic acid | 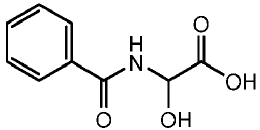 |
| Butyric acid | 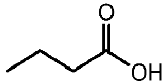 |
| 2-Ethylmalonic acid | 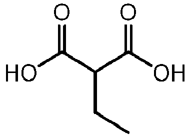 |
| Glutamine | 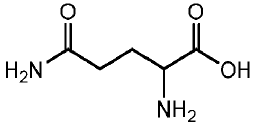 |
| 2-Hydroxy-2-phenylacetic acid | 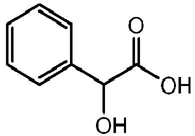 |
| 2-Oxo-3-phenylpropanoic acid | 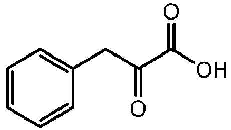 |
| 4-(aminomethyl)-2,6-difluorophenol | 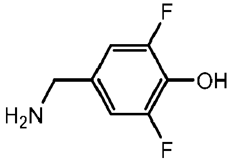 |
| 3-(aminomethyl)-2,6-difluorophenol | 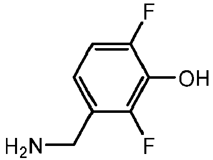 |
| 4-aminobutanoic acid | 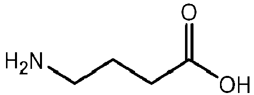 |
| 5-Oxohexanoic acid | 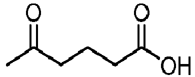 |
| 3-Hydroxy-3-methylbutanoic acid | 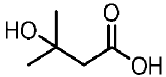 |
| 3-Methyl-2-oxopentanoic acid | 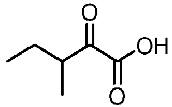 |
| (3-Methylbenzoyl)glycine | 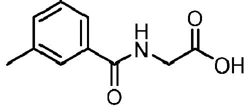 |
| 3-Methylpentanoic acid | 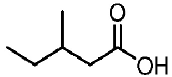 |
| (4-Methylbenzoyl)glycine | 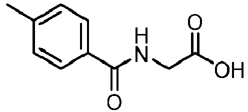 |
| Hexanedioic acid | 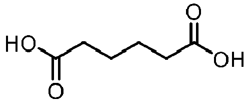 |
| Dimethylmalonic acid | 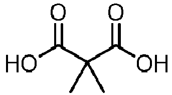 |
| Alanine | 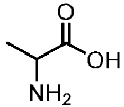 |
| Fumaric acid | 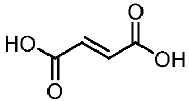 |
| Glutamic acid | 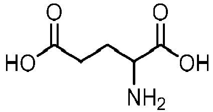 |
| Glutaric acid | 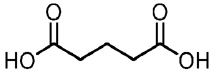 |
| Glycine | 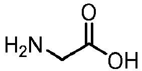 |
| 2-(1H-indol-3-yl)acetic acid | 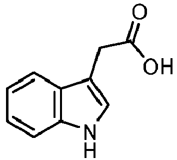 |
| 2-isopropylmalonic acid | 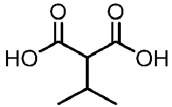 |
| 4-hydroxyquinoline-2-carboxylic acid | 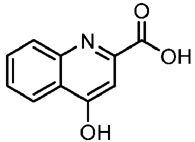 |
| Asparagine | 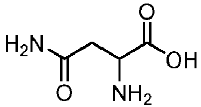 |
| Isoleucine | 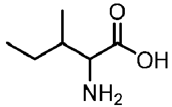 |
| Tartaric acid | 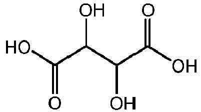 |
| Valine | 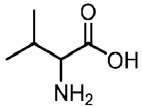 |
| 4-oxopentanoic acid | 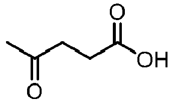 |
| Lysine | 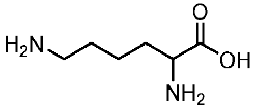 |
| 2-Oxoglutaric acid | 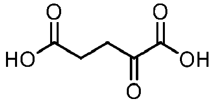 |
| Proline | 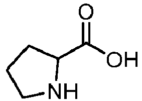 |
| Quinoline-2-carboxylic acid | 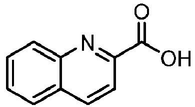 |
| Serine | 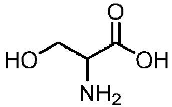 |
| Octanedioic acid | 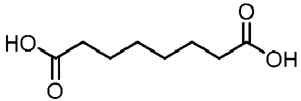 |
| Succinic acid | 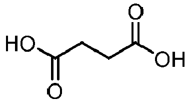 |
| Tryptophan | 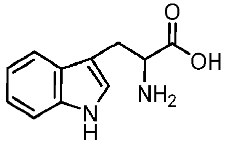 |
| 4-Methylvaleric acid | 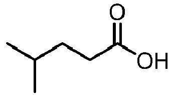 |
| 2-Oxosuccinic acid | 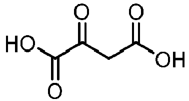 |
| Methylsuccinic acid | 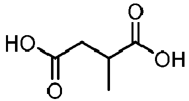 |
| 2-hydroxybutyric acid | 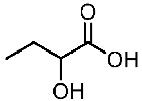 |
| Choline | 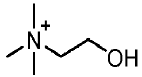 |
| Guanidine | 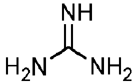 |
| Arginine | 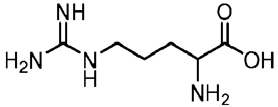 |
| 3-(((9Z, 12Z)-octadeca-9,12-dienoyl)oxy)propane-1,2-diylbis(3-hydroxybutanoate) | 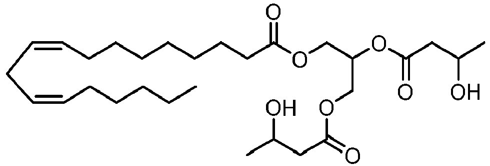 |
| 3 -((4-aminobutanoyl)oxy)-2-((3 - hydroxybutanoyl)oxy)propyl (9Z,12Z)-octadeca-9,12-dienoate | 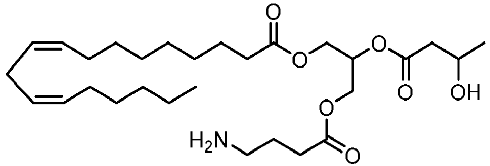 |
| 3 -(((9Z, 12Z)-octadeca-9,12-dienoyl)oxy)propane-1,2-diylbis(2,5-diamino-5-oxopentanoate) | 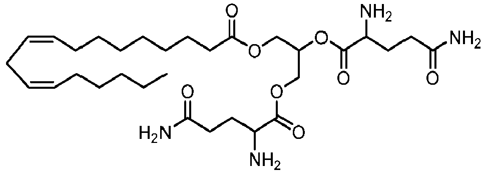 |
| 2-((4-aminobutanoyl)oxy)-3-(glutaminyloxy)propyl (9Z,12Z)-octadeca-9,12-dienoate | 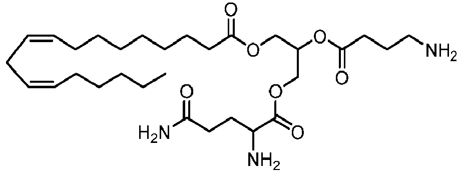 |

In some embodiments, a pharmaceutical composition disclosed herein may comprise a therapeutically-effective amount of a compound selected from: 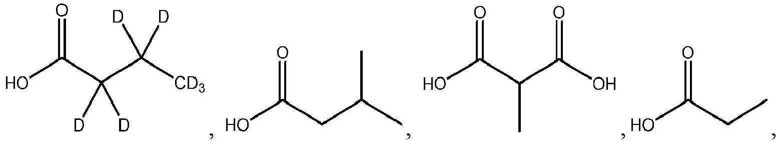 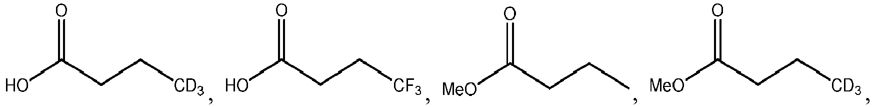 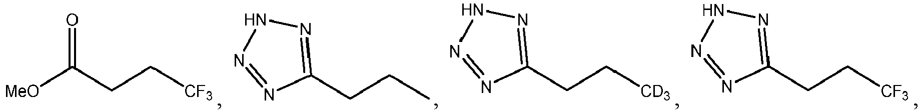 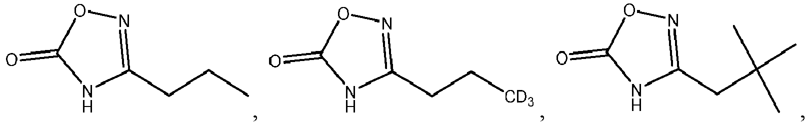 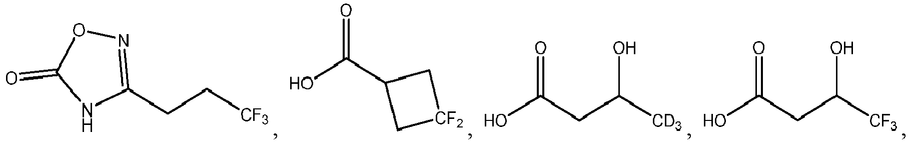 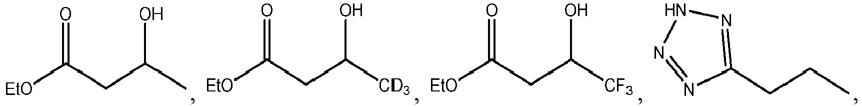 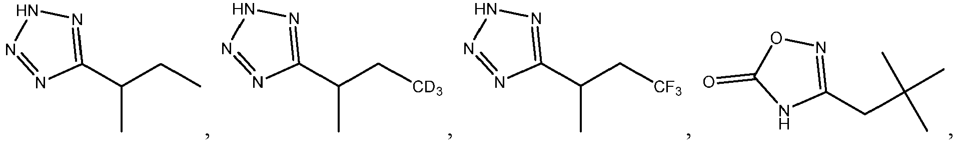 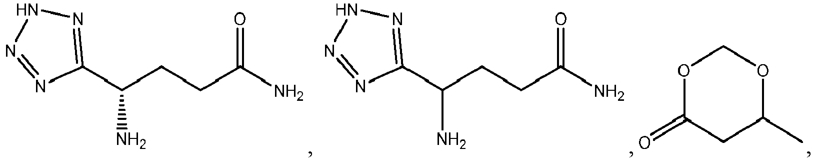 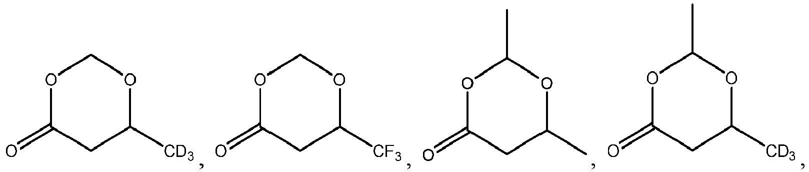 and 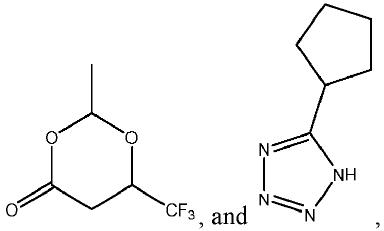 or a salt of any one thereof.

In some embodiments, a pharmaceutical composition disclosed herein may comprise a therapeutically-effective amount of a compound selected from: 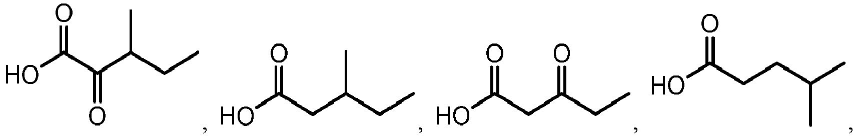 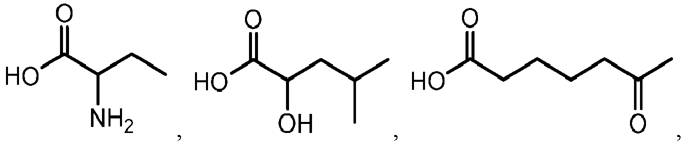 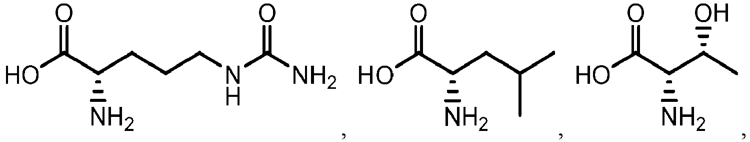 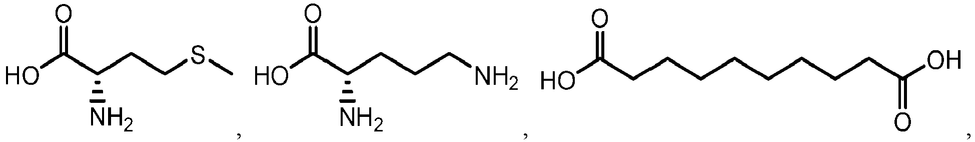 or a salt of any one thereof.

In some embodiments, a pharmaceutical composition disclosed herein may comprise a therapeutically-effective amount of a compound selected from: isoflurane, halothane, ethanol, octanol, dodecanol, sodium octyl sulfate (SOS), octadecyltrimethylammonium bromide (OTABr), sodium dodecyl sulfate (SDS), 2,5-diacetoxyphenyl sulfonate (DAPS), dodecyl trimethyl ammonium chloride (DATCl), or a salt of any one thereof.

In some embodiments, a pharmaceutical composition comprises a therapeutically-effective amount of (S)-3-hydroxybutanoic acid, (R)-3-hydroxybutanoic acid, 3,3-dimethylbutanoic acid, 2-benzamido-2-hydroxyacetic acid, butyric acid, 2-ethylmalonic acid, glutamine, 2-hydroxy-2-phenylacetic acid, 2-oxo-3-phenylpropanoic acid, 4-(aminomethyl)-2,6-difluorophenol, 3-(aminomethyl)-2,6-difluorophenol, 4-aminobutanoic acid, 5-oxohexanoic acid, 3-hydroxy-3-methylbutanoic acid, 3-methyl-2-oxopentanoic acid, (3-methylbenzoyl)glycine, 3-methylpentanoic acid, (4-methylbenzoyl)glycine, hexanedioic acid, dimethylmalonic acid, alanine, fumaric acid, glutamic acid, glutaric acid, glycine, 2-(1h-indol-3-yl)acetic acid, 2-isopropylmalonic acid, 4-hydroxyquinoline-2-carboxylic acid, asparagine, isoleucine, tartaric acid, valine, 4-oxopentanoic acid, lysine, 2-oxoglutaric acid, proline, quinoline-2-carboxylic acid, serine, octanedioic acid, succinic acid, tryptophan, 4-methylvaleric acid, 2-oxosuccinic acid, methylsuccinic acid, 2-hydroxybutyric acid, choline, guanidine, arginine, 3-(((9Z,12Z)-octadeca-9,12-dienoyl)oxy)propane-1,2-diyl bis(3-hydroxybutanoate), 3-((4-aminobutanoyl)oxy)-2-((3-hydroxybutanoyl)oxy)propyl (9Z,12Z)-octadeca-9,12-dienoate, 3-(((9Z,12Z)-octadeca-9,12-dienoyl)oxy)propane-1,2-diyl bis(2,5-diamino-5-oxopentanoate), 2-((4-aminobutanoyl)oxy)-3-(glutaminyloxy)propyl (9Z,12Z)-octadeca-9,12-dienoate, 5-cyclopentyl-1H-tetrazole, or a salt thereof.

In some cases, a pharmaceutical composition comprises (S)-3-hydroxybutanoic acid, (R)-3-hydroxybutanoic acid, 3,3-dimethylbutanoic acid, 2-benzamido-2-hydroxyacetic acid, butyric acid, 2-ethylmalonic acid, glutamine, ethylmalonic acid, or a salt thereof. In some cases, a pharmaceutical composition comprises 3-hydroxybutyric acid, butyric acid, ethylmalonic acid, or a salt thereof.

In some cases, a pharmaceutical composition comprises ethyl malonic acid, S-3-hydroxybutyric acid, r-3-hydroxybutyric acid, butyric acid, mandelic acid, glutamine, 3,3-dimethyl butyric acid, αlpha-hydroxyhippuric acid, phenylpyruvic acid, 3-hydroxy-3-methylbutyric acid, l-valine, 2-oxoglutaric acid, 4-methylvaleric acid, kynurenic acid, indole-3-acetic acid, 3-methylhippuric acid, l-tartaric acid, proline, 3-methylvaleric acid, fumaric acid, isopropyl malonic acid, choline, glutaric acid, arginine , tryptophan, 4-acetylbutyric acid, 4-methylhippuric acid, l-serine, isoleucine, dimethylmalonic acid, succinic acid, guanidine, adipic acid, quinaldic acid, suberic acid, l-asparagine, 2-hydroxybutyric acid, 3-methy-12-oxovaleric acid, levulinic acid, glycine, lysine, oxaloacetic acid, alanine, methylsuccinic acid, glutamate, or a salt thereof.

In some cases, a compound of the current disclosure may be described as a surfactant, wherein it comprises a polar moiety and a non-polar or less polar moiety. In some cases, a compound of the current disclosure may be used to form a dimer, trimer, or polymer. In some cases, a compound of the current disclosure may be used to form a gemini surfactant.

In some embodiments, a pharmaceutical composition disclosed herein may comprise a therapeutically-effective amount of a compound that is a surfactant. In some embodiments, a pharmaceutical composition disclosed herein may comprise a therapeutically-effective amount of a compound that is derived from, made from, or synthesized from a surfactant.

A surfactant may be a compound that is amphiphilic, wherein the compound contains both hydrophobic groups and hydrophilic groups. The surfactant may contain both a water-insoluble, or oil-soluble, group and a water-soluble group.

In some embodiments, a surfactant may be nonionic, anionic, ampholytic, zwitterionic, cationic, or a combination thereof.

Nonionic surfactants may be polyethyleneoxide condensates of alkyl phenols or the condensation products of aliphatic alcohols with from about 1 to about 25 moles of ethylene oxide. Anionic surfactants may be alkali metal soaps such as the sodium, potassium, ammonium and alkylolammonium salts of higher fatty acids containing from about 8 to about 24 carbon atoms, or water-soluble salts, such as alkali metal, ammonium and alkylolammonium salts, of organic sulfuric reaction products having in their molecular structure an alkyl group containing from about 10 to about 20 carbon atoms and a sulfonic acid or sulfuric acid ester group.

Ampholytic surfactants may be aliphatic derivatives of secondary or tertiary amines, or aliphatic derivatives of heterocyclic secondary and tertiary amines in which the aliphatic radical can be straight chain or branched and wherein one of the aliphatic substituents contains from about 8 to 18 carbon atoms and at least one contains an anionic water-solubilizing group.

Zwitterionic surfactants can be derivatives of secondary and tertiary amines, derivatives of heterocyclic secondary and tertiary amines.

Cationic surfactants may be quaternary ammonium surfactants.

Examples of surfactants include docusate (dioctyl sodium sulfosuccinate), perfluorooctanesulfonate (PFOS), perfluorobutanesulfonate, alkyl-aryl ether phosphates, alkyl ether phosphates, cetrimonium bromide (CTAB), cetylpyridinium chloride (CPC), benzalkonium chloride (BAC), benzethonium chloride (BZT), dimethyldioctadecylammonium chloride, dioctadecyldimethylammonium bromide (DODAB), octaethylene glycol monododecyl ether, pentaethylene glycol monododecyl ether, sorbitan monolaurate, sorbitan monostearate, sorbitan tristearate, phosphine oxide, dimethyl sulfoxide, sodium and potassium salts of ethylene-1,1-diphosphonic acid, the sodium and potassium salts of ethane 1-hydroxy-1,1-diphosphonic acid and the sodium and potassium salts of ethane, 1,1,2-triphosphonic acid. sodium and potassium carbonate, bicarbonate, sesquicarbonate, and tetraborate decahydrate.

A compound may be used to form a dimer, wherein the monomers are connected via a covalent bond. A compound may be used to form a dimer, wherein the monomers are connected through a linker. The linker may connect via a polar group on the monomer. In some cases, the polar group of a compound of the current disclosure may be a carbonyl group.

Non-limiting examples of linkers can include those which form an amide bond, an ester bond, an ether bond, a carbonate bond, a carbamate bond, or a thioether bond, and such functional groups on the linker can be, for example, amino groups; carboxyl groups; aldehyde groups; azide groups; alkyne and alkene groups; ketones; carbonates; carbonyl functionalities bonded to leaving groups such as cyano and succinimidyl and hydroxyl groups.

A linker moiety may be covalently bound to any position, valence permitting, on a compound or salt of Formula (I). For example, a linker may be bound to R¹, R², R³, R⁶, or R⁷.

In some cases, a sugar moiety may be attached to a compound or salt of the current disclosure. The sugar moiety may be a monosaccharide, a simple sugar, a disaccharide, a 5-carbon sugar, or a 6-carbon sugar.

Examples of a sugar include fructose, galactose, glucose, maltose, lactose, sucrose, and others. In some cases, a compound of the current disclosure may be linked to a fructose moiety, wherein the linkage may be via the polar end of the compound. In some cases, a compound of the current disclosure may be linked to a glucose moiety, wherein the linkage may be via the polar end of the compound.

A sugar moiety may be covalently bound to any position, valence permitting, on a compound or salt of Formula (I). For example, a sugar moiety may be bound to R¹, R², R³, R⁶, or R⁷.

In some cases, a nonpolar group can be attached to a compound of the current disclosure. Nonpolar groups include, for example, an alkyl group, an alkenyl group, an alkynyl group, an ethylene glycol group, a glycerol group, or a combination thereof.

In some cases, the effective amount of a compound or a salt thereof of this disclosure to be employed depends on the level of anesthesia to which the mammal is to be brought, the rate at which anesthesia is to be induced, and the length of time over which anesthesia is to be maintained. The amount used should be sufficient to provide a significant anesthetic effect but not so much as to produce unacceptable deleterious side effects. The amount of anesthesia to be used can be regulated, starting with a small amount of the compound and gradually increasing the amount until the desired plane of anesthesia is reached. By then monitoring the physical reactions of the mammal, as is the usual procedure, the duration and plane of anesthesia can be readily controlled.

The amount of each compound administered will be dependent on the mammal being treated, the severity of the disorder or condition, the rate of administration, the disposition of the compound and the discretion of the prescribing physician. However, an effective dosage may be in the range of about 0.001 to about 10,000 mg per kg body weight per day, in single or divided doses. In some instances, dosage levels below the lower limit of the aforesaid range may be more than adequate, while in other cases still larger doses may be employed without causing any harmful side effect, e.g., by dividing such larger doses into several small doses for administration throughout the day.

In some embodiments, the disclosure provides a method for administration of a compound of the current disclosure to a subject in need thereof. In some embodiments, a pharmaceutical composition comprising a compound of the current disclosure is administered to a subject in need thereof.

Subjects may be monitored for therapeutic effectiveness using assays and methods suitable for the condition being treated, which assays will be familiar to those having ordinary skill in the art and are described herein. Pharmacokinetics of a compound or a salt thereof of the current disclosure that is administered to a subject may be monitored by determining the level of the compound in a biological fluid, for example, in the blood, blood fraction (e.g., serum), in the urine, in expired air, or other biological sample or biological tissue from the subject. Any method practiced in the art and described herein to detect the compound may be used to measure the level of the compound during a treatment course.

In some embodiments, the pharmaceutical composition is administered to a patient in a unit dose. A unit dose that is administered to a patient may comprise from about 0.0001-500 g, 0.001-250 g, 0.01-100 g, 0.1-50 g, or 1 - 10 g of a compound or a salt thereof of the current disclosure. In some embodiments, the pharmaceutical composition comprises about or more than about 0.0001 g, 0.001 g, 0.01g, 0.1, 0.5 g, 1 g, 2 g, 3 g, 4 g, 5 g, 6 g, 7 g, 8 g, 9 g, 10 g, 15 g, 20 g, 25 g, 50g, 100 g, 200 g, 250 g, 300 g, 350 g, 400 g, 450 g, 500 g, or more of a compound or a salt thereof of the current disclosure. In some embodiments, the pharmaceutical composition comprises from 0.001 - 2 g of a compound or a salt thereof of the current disclosure in a single dose. In some embodiments, the pharmaceutical composition comprises an amount between about 50-150 g of a compound or a salt thereof of the current disclosure. In some embodiments, the therapeutic amount can be an amount from about 0.001-0.1 g of a compound or a salt thereof of the current disclosure. In some embodiments, the therapeutic amount can be an amount from about 0.01-30 g of a compound or a salt thereof of the current disclosure.

In some embodiments, a therapeutically effective amount of a compound or a salt thereof of the current disclosure, which can be an amount administered over a certain period, can sufficiently provide any one or more of the therapeutic effects described herein. As an example, the therapeutic effective amount can be in the range of about 0.001-1000 mg/kg body weight, 0.01-500 mg/kg body weight, 0.01-100 mg/kg body weight, 0.01-30 mg/kg body weight, 0.1-200 mg/kg body weight, 3-200 mg/kg body weight, 5 - 500 mg/kg body weight, 10 - 100 mg/kg body weight, 10 - 1000 mg/kg body weight, 50- 200 mg/kg body weight, 100- 1000 mg/kg body weight, 200 - 500 mg/kg body weight, 250-350 mg/kg body weight, or 300 - 600 mg/kg body weight of a compound or a salt thereof of the current disclosure. In some embodiments, the effective amount is at least about 0.01 mg/kg body weight of a compound or a salt thereof of the current disclosure. In some embodiments, the effective amount is an amount from about 0.01 - 30 mg/kg body weight of a compound or a salt thereof of the current disclosure. In some embodiments, the therapeutic amount can be an amount from about 50-150 mg/kg body weight of a compound or a salt thereof of the current disclosure.

In some embodiments, a therapeutically effective amount of a compound or a salt thereof of the current disclosure, which can be an amount administered over a certain period, can sufficiently provide any one or more of the therapeutic effects described herein. As an example, the therapeutic effective amount can be formulated into a unit dose, wherein the unit dose may have a concentration in the range of about 0.001-1000 mg/mL, 0.01-500 mg/mL, 0.01-100 mg/mL, 0.01-30 mg/mL, 0.1- 200 mg/mL, 1-200 mg/mL, 5 - 500 mg/mL, 10 - 100 mg/mL, 10 - 1000 mg/mL, 50- 200 mg/mL, 100- 1000 mg/mL, 200 - 500 mg/mL, 250-350 mg/mL, or 300 - 600 mg/mL.

In some embodiments, the composition is provided in one or more unit doses. For example, the composition can be administered in 1, 2, 3, 4, 5, 6, 7, 14, 30, 60, or more doses. Such amount can be administered each day, for example in individual doses administered once, twice, or three or more times a day. However, dosages stated herein on a per day basis should not be construed to require administration of the daily dose each and every day. For example, if one of the agents is provided in a suitably slow-release form, two or more daily dosage amounts can be administered at a lower frequency, e.g., as a depot every second day to once a month or even longer. In some cases, a unit dose may be administered daily. In some cases, a unit dose may be administered every other day.

A unit dose of a compound or a salt thereof of the current disclosure may be administered as a solution and may have a certain concentration or molarity. In some cases, a unit dose may have a molarity at least about 0.01 molar (M), 0.05 M, 0.1 M, 0.2 M, 0.3 M, 0.4 M, 0.5 M, 0.6 M, 0.625 M, 0.7 M, 0.8 M, 0.9 M, 1 M, 1.1 M, 1.2 M, 1.3 M, 1.4 M, 1.5 M, 1.6 M, 1.7 M, 1.8 M, 1.9 M, 2 M, 3 M, 4 M, 5 M, 6 M, 7 M, 8 M, 9 M, 10 M, 15 M, 20 M, or 25 M. In some cases, a unit dose may have a molarity of about 1 M, 1.5 M, 2 M, 3M, 4 M, or 5M. In some cases, a unit dose may have a molarity of about 1 M, 1.5 M, 2 M, or 3M. In some cases, a unit dose may have a molarity of about 2 M. In some cases, a unit dose may have a molarity of about 1.5 M. In some cases, a unit dose may have a molarity of about 0.1 M, 0.2 M, 0.3 M, 0.4 M, 0.5 M, 0.6 M, or 0.7 M. A unit dose of a compound or a salt thereof of the current disclosure may be a solution wherein the volume of the dose is at least about 0.1 mL, 0.2 mL, 0.3 mL, 0.4 mL, 0.5 mL, 0.6 mL, 0.7 mL, 0.8 mL, 0.9 mL, 1 mL, 2 mL, 5 mL, 10 mL, or 20 mL.

The unit doses can be administered simultaneously or sequentially. The composition can be administered for an extended treatment period. Illustratively, the treatment period can be at least about one month, for example at least about 3 months, at least about 6 months or at least about 1 year. In some cases, administration can continue for substantially the remainder of the life of the subject.

The term "about" when referring to a number or a numerical range means that the number or numerical range referred to is an approximation within experimental variability (or within statistical experimental error), and thus the number or numerical range may vary from, for example, between 1% and 10% of the stated number or numerical range.

In certain particular embodiments, more than one compound or a salt thereof of the current disclosure may be administered at a time to a subject. In some embodiments, two compounds of the current disclosure in combination make act synergistically or additively, and either compound may be used in a lesser amount than if administered alone.

In some embodiments, a compound or a salt thereof of the current disclosure may be a prodrug.

In some embodiments herein, one or more of any compounds or salts thereof disclosed herein may be excluded from the claims.

Solutions of varying molarity of a compound of Formula I can be prepared. In some cases, solutions may be administered subcutaneously. A subject may become anesthetized and/or lose consciousness for a certain period of time, and wake up a certain period of time later.

### Combination Therapy

In certain embodiments, a compound or a salt thereof disclosed herein and/or pharmaceutical compositions thereof can be used in combination therapy with other therapeutic agents. A compound or a salt thereof disclosed herein and/or pharmaceutical compositions thereof and the therapeutic agent can act additively or, more preferably, synergistically. In some embodiments, a compound or a salt thereof disclosed herein and/or pharmaceutical compositions thereof are administered concurrently with the administration of another therapeutic agent. For example, a compound or a salt thereof disclosed herein and/or pharmaceutical compositions thereof may be administered together with another therapeutic agent. In other embodiments, a compound or a salt thereof disclosed herein and/or pharmaceutical compositions thereof are administered prior or subsequent to administration of other therapeutic agents.

In some embodiments, a compound or a salt thereof disclosed herein may be used in combination with an enzyme inhibitor. An enzyme inhibitor may bind to an enzyme and at least partially inhibit or decrease its activity. While not wishing to be bound by theory, an enzyme inhibitor may reduce the rate at which a compound, a salt thereof, or a metabolite thereof, of the current disclosure is eliminated or metabolized in a subject.

In some embodiments, a compound or a salt thereof of the current disclosure may be used in combination with an enzyme inhibitor selected from: 3-methyl-2-oxobutanoate dehydrogenase, 3-methyl-2-oxobutanoate dehydrogenase, methylmalonyl-CoA mutase, arginase, amino-acid N-acetyltransferase, 3-oxoacid CoA-transferase, alcohol dehydrogenase, short-chain acyl-CoA dehydrogenase, and any combination thereof.

In some embodiments, an enzyme inhibitor may be selected from: 2-chloro-4-methylpentanoate,2-chloroisocaproate, 2-oxobutanoate, 2-oxoglutarate, 2-oxopentanoate, 3-methyl-2-oxobutanoate, 3-methyl-2-oxopentanoate, 3-methylbutanoyl-CoA, 4-(2-thienyl)-2-oxo-3-butenoate, 4-(3-thienyl)-2-oxo-3-butenoate, 4-methyl-2-oxopentanoate, alpha-ketoisocaproate, cinnamylpyruvate, D-3-methyl-2-oxopentanoate, furfurylidenepyruvate, L-3-methyl-2-oxopentanoate, and a salt of any one thereof.

In some embodiments, an enzyme inhibitor may be selected from: 2-(N-morpholino)propane sulfonate buffer, 2-chloroisohexanoate, 2-oxo-3-methylpentanoate, 2-oxobutanoate, 2-oxohexanedioate, 2-oxohexanoate, 2-oxoisocaproate, 2-oxoisopentanoate, 2-oxopentanoate, 3-methyl-2-oxobutanoate, 4-(2-thienyl)-2-oxo-3-butenoate, 4-(3-thienyl)-2-oxo-3-butenoate, 4-hydroxyphenylacetate, 4-hydroxyphenyllactate, 4-hydroxyphenylpyruvate, 4-methyl-2-oxopentanoate, alpha-chloroisocaproate, alpha-ketoisocaproate, alpha-ketoisovalerate, branched-chain 2-oxo acids, clofibric acid, dichloroacetate, furfurylidenepyruvate, N-octanoate, phenylacetate, phenylpyruvate, and a salt of any one thereof.

In some embodiments, an enzyme inhibitor may be selected from: 2-(N,N-diethylamino)-diazenolate-2-oxide, 5,5'-dithiobis(2-nitrobenzoic acid), p-chloromercuribenzoate, p-hydroxymercuribenzoate, and a salt of any one thereof.

In some embodiments, an enzyme inhibitor may be selected from: (-)-epicatechin, (2S)-2-amino-3-(2-amino-1H-imidazol-5-yl)propanoic acid, (2S)-2-amino-5-(1H-imidazol-2-ylamino)pentanoic acid, (2S,5E)-2-amino-7-oxohept-5-enoic acid, (R)-2-amino-6-borono-2-[2-(piperidin-1-yl)ethyl]hexanoic acid, (R)-2-amino-6-borono—2[1-(3,4-dichlorobenzyl)piperidin-4-yl]hexanoic acid, (S)-(2-boronoethyl)-L-cysteine, (S)-2-amino-7-oxoheptanoic acid, 2(S)-amino-6-boronohexanoic acid, 2-(1H-indol-3-yl)ethanol, 2-(5-methyl-2-(trifluoromethyl)-[1,2,4]triazolo[1,5-a]pyrimidin-7-yl)hydrazinecarbothioamide, 2-(aminomethyl)-6-borononorleucine, 2-(S)-amino-5-(2-aminoimidazol-1-yl)pentanoic acid, 2-(S)-amino-6-boronohexanoic acid, 2-amino-6-borono-2-(difluoromethyl)-hexanoic acid, 2-amino-6-borono-2-butylhexanoic acid, 2-amino-6-borono-2-methyl-hexanoic acid, 2-amino-6-boronohexanoic acid, 2-mercaptoethanol, 2-mercaptopropionate, 2-oxoarginine, 2-[(benzylamino)methyl]-6-borononorleucine, 3-mercaptopropionate, 5-hydroxy-L-tryptophan, 5-hydroxytryptamine, 5-methyl-2-(trifluoromethyl)-N-[3-(trifluoromethyl)-1H-1,2,4-triazol-5-yl][1,2,4]triazolo[1,5-a]pyrimidin-7-amine, 5-methyl-7-(1H-1,2,4-triazol-1-yl)-2-(trifluoromethyl)[1,2,4]triazolo[1,5-a]pyrimidine, 5-methyl-7-(1H-pyrrol-1-yl)-2-(trifluoromethyl)[1,2,4]triazolo[1,5-a]pyrimidine, 5-methyl-7-(4-trifluorophenylamine)-2-(trifluoromethyl)[1,2,4]triazolo[1,5-a]pyrimidine, 5-methyl-7-(pyrrolidin-1-yl)-2-(trifluoromethyl)[1,2,4]triazolo[1,5-a]pyrimidine, 5-methyl-N-(naphthalen-2-yl)-2-(trifluoromethyl)[1,2,4]triazolo[1,5-a]pyrimidin-7-amine, 6-borono-2-(2-hydroxyethyl)norleucine, 6-borono-2-(3-methoxypropyl)norleucine, 6-borono-2-(hydroxymethyl)norleucine, 6-borono-2-ethylnorleucine, 6-borono-2-methylnorleucine, 6-borono-2-propan-2-ylnorleucine, 6-borono-2-[2-(4-hydroxypiperidin-1-yl)ethyl]norleucine, 6-borono-2-[2-(diethylamino)ethyl]norleucine, 6-borono-2-[2-(morpholin-4-yl)ethyl]norleucine, 6-borono-2-[2-(pyrrolidin-1-yl)ethyl]norleucine, 7-(4-chlorophenylamine)-5-methyl-2-(trifluoromethyl)[1,2,4]triazolo[1,5-a]pyrimidine, acetyl hydroxamate, agmatine, alpha-(4-boronobutyl)histidine, alpha-(4-boronobutyl)phenylalanine, chloroquine, cycloheximide, D-arginine, D-tryptophan, diethyl dicarbonate, diethylene triamine-nitric oxide, dithiothreitol, homocysteine, edeine B1, gamma-guanidinobutyrate, glutathione, guanidinium chloride, guanidinoacetic acid, hydroxylamine, imidazole-3-lactate, indole, indole-3-L-lactic acid, indolepropionic acid, indolepropionic acid, indospicine, inosine, iodoacetamide, L-2-amino-3-guanidinopropionic acid, L-argininamide, L-Argininic acid, L-canavanine, L-homoarginine, L-isoleucine, L-N5-(1-iminoethyl)-ornithine, L-N6-(1-iminoethyl)-lysine, L-norvaline, L-proline, L-thiocitrulline, L-tryptophan, L-valine, lysine, N-(2,6-difluorophenyl)-5-methyl-2-(trifluoromethyl)[1,2,4]triazolo[1,5-a]pyrimidin-7-amine,N-(3,4-dichlorophenyl)-5-methyl-2-(trifluoromethyl)[1,2,4]triazolo[1,5-a]pyrimidin-7-amine,N-(3,5-dichlorophenyl)-2,5-bis(trifluoromethyl)[1,2,4]triazolo[1,5-a]pyrimidin-7-amine,N-(3,5-dichlorophenyl)-2,5-dimethyl[1,2,4]triazolo[1,5-a]pyrimidin-7-amine, N-(3,5-dichlorophenyl)-5-methyl-2-(trifluoromethyl)[1,2,4]triazolo[1,5-a]pyrimidin-7-amine,N-(3,5-dimethoxyphenyl)-5-methyl-2-(trifluoromethyl)[1,2,4]triazolo[1,5-a]pyrimidin-7-amine,N-(3-chlorophenyl)-5-methyl-2-(trifluoromethyl)[1,2,4]triazolo[1,5-a]pyrimidin-7-amine, N-(4-methoxyphenyl)-5-methyl-2-(trifluoromethyl)[1,2,4]triazolo[1,5-a]pyrimidin-7-amine, N-acetyl-5-hydroxytryptamine, N-bromosuccinimide, N-hydroxy-nor-L-arginine, N-hydroxyl-L-arginine, Nomega-amino-L-arginine, nomega-hydroxy-L-arginine, nomega-hydroxy-nor-arginine, nomega-hydroxy-nor-L-arginine, nomega-nitro-L-arginine, nomega-nitro-L-arginine methyl ester, nor-N-hydroxy-L-arginine, nor-NOHA, NOHA, nor-nomega-hydroxy-L-arginine, propargylglycine, S-(2-aminoethyl)isothiourea, S-(2-boronoethyl)-L-cysteine, S-(2-boronomethyl)-L-cysteine, S-(2-boronomethyl)-L-homocysteine, S-(3-aminopropyl)isothiourea, S-(boronoethyl)-L-cysteine, S-methyl-L-thiocitrulline, S-nitrosoglutathione, sodium nitroprusside, tryptamine, tyramine, and a salt of any one thereof.

In some embodiments, an enzyme inhibitor may be selected from: 1,3-diaminopropane, 5,5'-dithiobis(2-nitrobenzoate), isobutylmethylxanthine, L-alpha-acetoxylglutamate, L-indospicine, N-acetyl-D-glutamate, N-acetyl-DL-alpha-aminoadipate, N-acetyl-L-aspartate, N-acetyl-L-glutamate, N-acetyl-L-glutamine, N-acetylglutamate, N-benzoyl-L-glutamate, N-butyryl-L-glutamate, N-carbamoyl-L-glutamate, N-ethylmaleimide, N-propionyl-L-glutamate, O-(L-norvalyl-5)-isourea, spermidine, spermine, and a salt of any one thereof.

In some embodiments, an enzyme inhibitor may be selected from: 2,2-difluorosuccinate, 2,4-Dinitrophenylacetate, 2-Nitro-5-(thiocyanato)benzoate, 3-sulfopropanoate, 4-nitrophenylacetate, 5,5'-dithiobis(2-nitrobenzoic acid), acetylimidazole, desulfopantetheine, dtnb, glutarate, iodoacetamide, malate, maleamate, maleimide, monomethylsuccinate, N-acetylaletheine, N-acetylcysteamine, N-ethylmaleamate, N-ethylmaleimide, pantothenol, perfluorosuccinate, succinamate, and a salt of any one thereof.

In some embodiments, an enzyme inhibitor may be selected from: 1,10-phenanthroline, 1,2-dithioglycerol, 1,2-ethanedithiol, 1,3-propanedithiol, 1,4-butanedithiol, 1,4-dioxane, 1,4-dithioerythritol, 1,4-dithiothreitol, 1-butyl-3-methylimidazolium tetrafluoroborate, 1-hydroxypyridine-2-thione, 1-thio-1-phenylmethane, 1-thioacetamide, 1-thioacetate, 1-thiobutane, 1-thioethane, 1-thioglycerol, 1-thiopropane, 1-thiosorbitol, 12-hydroxydodecanoate, 2,2'-bipyridine, 2,2'-bipyridyl, 2,2'-dipyridyl, 2,2,2-trifluoroethanol, 2,4-dinitrophenol, 2-chloroethanol, 2-fluoroethanol, 2-mercapto-1-methylimidazole, 2-mercaptobenzimidazole, 2-mercaptobenzothiazole, 2-mercaptoethanol, 2-mercaptoimidazole, 2-phenylethanethiol, 2-pyridylethanethiol, 2-thioacetate, 2-thiobutane, 2-thiopropane, 2-thiopyridine, 2-thiopyrimidine, 3-butylthiolan 1-oxide, 3-mercapto-1,2,4-triazole, 3-thiopropionate, 4-androsten-3,17-dione, 4-bromopyrazole, 4-cyanopyrazole, 4-iodopyrazole, 4-methoxypyrazole, 4-Methylpyrazole, 4-nitropyrazole, 4-octylpyrazole, 4-pentylpyrazole, 4-propylpyrazole, 5-beta-D-ribofuranosylnicotinamide adenine dinucleotide, 5alpha-androstan-17beta-ol-3-one, 6-thioguanine, 6-thioguanosine, 8-amino-6-methoxyquinoline, 8-hydroxyquinoline 5-sulfonic acid, acetamide, antimycin, butyramide, caffeic acid, captopril, cyclobutyl carbinol, cyclohexylformamide, cyclopropyl carbinol, cysteamine, diethyldithiocarbamate, dipicolinic acid, dodecanoic acid, ellagic acid, genistein, guanidine hydrochloride, heptafluorobutanol, heptane, hexadecane, hexadecyltrimethyl-ammonium bromide, Hydroxylamine hydrochloride, imidazole, iodoacetamide, iodoacetate, iodoacetic acid, isoburyramide, Isobutyramide, isooctane, mithramycin, N-1-methylheptylformamide, N-benzylformamide, N-cyclopentyl-N-cyclobutylformamide, N-ethylmaleimide, N-heptylformamide, o-phenanthroline, Octanoic acid, p-chloromercuribenzene sulfonate, p-hydroxymercuribenzoate, Penicillamine, pefabloc, polyoxyethylene octylphenyl ether, pyrazole, Pyridine, pyridoxal 5'-phosphate, quercetin, S-2-chloro-3-(imidazol-5-yl)propionate, syringaldehyde, tert-butanol, tert-butyl hydroperoxide, thiophenol, thiourea, toluene, trichloroethanol, trifluoroethanol, vanillin, and a salt of any one thereof.

In some embodiments, an enzyme inhibitor may be selected from: 1-azepan-1-yl-2-phenyl-2-(4-thioxo-1,4-dihydro-pyrazolo[3,4-d]pyrimidin-5-yl)-ethanone, 3-Chloro-3-butenoylpantetheine, 3-Pentenoylpantetheine, 4-chloromercuribenzoate, diethyl dicarbonate, iodoacetamide, N-ethylmaleimide, p-hydroxymercuribenzoate, and a salt of any one thereof.

In some embodiments, a compound or a salt thereof of the current disclosure may be dosed in combination with another class of drugs, including, but not limited to, antidepressants, tricyclic antidepressants, amiodarone, antihistamines, beta adrenergic agonists, cyclosporine a, depakote, lamotrigine, lithium, metoclopramide, monoamine oxidase inhibitors, neuroleptics, nicotine, nifedipine, theophylline, thyroid hormones, valproic acid, and any combination thereof.

### Formulations

When desired, the (R)- and (S)-isomers of the compounds, or salts thereof, of the present disclosure, if present, may be resolved by methods known to those skilled in the art, for example by formation of diastereoisomeric salts or complexes which may be separated, for example, by crystallization; via formation of diasteroisomeric derivatives which may be separated, for example, by crystallization, gas-liquid or liquid chromatography; selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. Alternatively, a specific enantiomer may be synthesized by asymmetric synthesis using optically active reagents, substrates, catalysts or solvents, or by converting one enantiomer to the other by asymmetric transformation.

When employed as pharmaceuticals, a compound or a salt thereof described herein can be administered in the form of pharmaceutical compositions. This disclosure therefore provides pharmaceutical compositions which contain, as the active ingredient, one or more of the compounds of Formula I or a pharmaceutically acceptable salt thereof and one or more pharmaceutically acceptable excipients, carriers, diluents, permeation enhancers, solubilizers and adjuvants. One or more compounds of Formula I may be administered alone or in combination with other therapeutic agents (e.g., vasoconstrictors, anti-inflammatory agents, antibiotics, other monobinding anesthetic bases and salts, counter-irritants), carriers, adjuvants, permeation enhancers, and the like. Pharmaceutically acceptable salts of the active agents (e.g., acid addition salts) may be prepared using standard procedures known to those skilled in the art of synthetic organic chemistry.

In some embodiments, a compound or a salt thereof of Formula I may be administered by any of the accepted modes of administration of agents having similar utilities, for example, by oral, topical, intradermal, intravenous, subcutaneous, intramuscular, intraarticular, intraspinal or spinal, epidural, rectal, vaginal, or transdermal/transmucosal routes. The most suitable route will depend on the nature and severity of the condition being treated. Subcutaneous, intradermal and percutaneous injections can be routes for a compound or a salt thereof of this disclosure. In some embodiments, a compound or a salt thereof of the current disclosure may be administered subcutaneously. In some embodiments, a compound or a salt thereof of the current disclosure may be administered intravenously. Sublingual administration may be a route of administration for a compound or a salt thereof of this disclosure. In making the compositions of this disclosure, the active ingredient can be diluted by an excipient. Some examples of suitable excipients include lactose, dextrose, sucrose, sorbitol, mannitol, starches, gum acacia, calcium phosphate, alginates, tragacanth, gelatin, calcium silicate, microcrystalline cellulose, PEG, polyvinylpyrrolidone, cellulose, water, sterile saline, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the disclosure can be formulated so as to provide quick, sustained or delayed release of the active ingredient after administration to the patient by employing procedures known in the art.

A pharmaceutical composition (e.g., for oral administration or for injection, infusion, subcutaneous delivery, intramuscular delivery, intraperitoneal delivery, sublingual delivery, or other method) may be in the form of a liquid. A liquid pharmaceutical composition may include, for example, one or more of the following: a sterile diluent such as water, saline solution, preferably physiological saline, Ringer's solution, isotonic sodium chloride, fixed oils that may serve as the solvent or suspending medium, polyethylene glycols, glycerin, propylene glycol or other solvents; antibacterial agents; antioxidants; chelating agents; buffers and agents for the adjustment of tonicity such as sodium chloride or dextrose. A parenteral composition can be enclosed in ampoules, disposable syringes or multiple dose vials made of glass or plastic. The use of physiological saline is preferred, and an injectable pharmaceutical composition is preferably sterile. In another embodiment, for treatment of an ophthalmological condition or disease, a liquid pharmaceutical composition may be applied to the eye in the form of eye drops. A liquid pharmaceutical composition may be delivered orally.

For oral formulations, at least one of the compounds described herein can be used alone or in combination with appropriate additives to make tablets, powders, granules or capsules, and if desired, with diluents, buffering agents, moistening agents, preservatives, coloring agents, and flavoring agents. A compound or a salt thereof may be formulated with a buffering agent to provide for protection of the compound from low pH of the gastric environment and/or an enteric coating. A compound included in a pharmaceutical composition may be formulated for oral delivery with a flavoring agent, *e*.*g*., in a liquid, solid or semi-solid formulation and/or with an enteric coating.

A pharmaceutical composition comprising any one of the compounds described herein may be formulated for sustained or slow release (also called timed release or controlled release). Such compositions may generally be prepared using well known technology and administered by, for example, oral, rectal, intradermal, or subcutaneous implantation, or by implantation at the desired target site. Sustained-release formulations may contain the compound dispersed in a carrier matrix and/or contained within a reservoir surrounded by a rate controlling membrane. Excipients for use within such formulations are biocompatible, and may also be biodegradable; preferably the formulation provides a relatively constant level of active component release. Non-limiting examples of excipients include water, alcohol, glycerol, chitosan, alginate, chondroitin, Vitamin E, mineral oil, and dimethyl sulfoxide (DMSO). The amount of compound contained within a sustained release formulation depends upon the site of implantation, the rate and expected duration of release, and the nature of the condition, disease or disorder to be treated or prevented.

Alternatively, a compound or a salt thereof of this disclosure may be solubilized and encapsulated (e.g., in a liposome or a biodegradable polymer), or used in the form of microcrystals coated with an appropriate nontoxic lipid. In some embodiments, a compound or a salt thereof of the current disclosure may be dissolved in intralipid before administration to a subject. The concentration of intralipid may be 10%, 20%, or 30%. The intralipid may be purchased or manufactured.

In some cases, the compositions may be formulated to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across tissue barriers.

In some cases, these compositions may be formulated as oral sprays. Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner. A nebulizer may be used to create a mist from a liquid dosage form.

For topical use, the compositions can be in the form of emulsions, creams, jelly, solutions, ointments containing, for example, up to 5% by weight of the active compound. For parenteral administration, the compositions can be in the form of sterile injectable solutions and sterile packaged powders.

Another formulation for use in the methods of the present disclosure employs transdermal delivery devices ("patches"). Such transdermal patches may be used to provide continuous or discontinuous infusion of a compound or a salt thereof of the present disclosure in controlled amounts. Such patches may be constructed for continuous, pulsatile, or on demand delivery of pharmaceutical agents.

The compositions are preferably formulated in a unit dosage form. The term unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., an ampoule).

A compound or a salt thereof described herein can be formulated as pharmaceutical compositions which are suitable for intravenous administration. For intravenous administration, a compound or a salt thereof of the present disclosure can be formulated in aqueous media using water-immiscible solvents, solubilizers, emulsifiers, surfactants or other solubilizing agents. Individual formulations may include one or more additional components such as stabilizers, tonicity modifiers, bases or acids to adjust pH, and solubilizers. The formulations can also optionally contain a preservative, such as ethylenediaminetetraacetic acid (EDTA) or sodium metabisulfate, to prevent the growth of microorganisms. Of course, any material used in preparing any unit dosage form should be pharmaceutically acceptable and substantially non-toxic in the amounts employed.

The pharmaceutical formulations can also include stabilizing agents, which can alternatively be considered as co-emulsifiers. Anionic stabilizers include phosphatidylethanolamines, conjugated with polyethylene glycol, (PEG-PE) and phosphatidylglycerols, a specific example of which is dimyristolphosphatidylgylcerol (DMPG). Additional examples of useful stabilizers include oleic acid and its sodium salt, cholic acid and deoxycholic acid and their respective salts, cationic lipids such as stearylamine and oleylamine, and 3β-[N-(N',N'-dimethylaminoethane)carbamoyl]cholesterol (DC-Chol).

The pharmaceutical compositions of the disclosure can be made isotonic with blood by the incorporation of a suitable tonicity modifier. Glycerol is most frequently used as a tonicity modifier. Alternative tonicity modifying agents include xylitol, mannitol, and sorbitol. The pharmaceutical compositions are typically formulated to be at physiologically neutral pH, typically in the range 6.0-8.5. The pH can be adjusted by the addition of base, for example sodium hydroxide or sodium bicarbonate, or in some cases acid, such as hydrochloric acid.

Solutions containing a compound or a salt thereof of present disclosure may be administered by injection or infusion, using suitable devices such as regular syringes or infusion devices, in the form of a pharmaceutical preparation which contains at least one compound or a salt thereof of the disclosure either as a free base or as a pharmaceutically acceptable, non-toxic acid addition salt, such as for example hydrochloride, lactate, acetate, sulfamate, in combination with a pharmaceutically acceptable carrier.

The concentration of active compound in a solution for injection may be from 0.01% to 10% by weight of the preparation.

Preferred solutions for injection or infusion or infiltration may be prepared as aqueous solutions of a water soluble, pharmaceutically acceptable salt of the active compound. These solutions may also contain stabilizing agents, antibacterial agents, buffering agents and may be manufactured in different dosage unit ampoules, single-use syringes or bottles. In any case, the quantity of the formulation containing the drug to be administered will be determined on an individual basis, and will be based on the pharmacological potency of the drug, the route of administration and at least in part on consideration of the individual's size, the severity of the symptoms to be treated and the results sought. In general, quantities of a compound or a salt thereof of the disclosure sufficient to eliminate the unwanted condition will be administered. The actual dosage (concentration and volume) and the number of administrations per day will depend on the pharmacokinetic properties of the drug and the mode of drug administrations, for example, for infiltration anesthesia of the skin.

In the present method, a compound or a salt thereof of the present disclosure can be administered topically to ocular mucous membranes of the eye or the mucous membranes surrounding the eye.

Formulations such as for example solutions, suspensions, gels or ointments may be useful.

Compatible carriers, which may be used in this disclosure, comprise e.g. an aqueous solution, such as saline solutions, oil solutions or ointments. Formulations for ocular use may also contain compatible and pharmaceutically acceptable excipients, such as preservatives, surfactants, stabilizing agents, antibacterial agents, buffering agents and agents such as for example polymers to adjust viscosity, vasoconstrictors, antihistaminic agents or anti-inflammatory agents. These formulations may be manufactured in different dosage units, suitable for ocular administration. Also drug inserts, either soluble or insoluble, may be used.

In the present method, a compound or a salt thereof of the present disclosure can be administered topically to non-ocular mucous membranes, such as for example oral, otic, nasal, respiratory, pharyngeal, tracheal, esophageal, urethral, or vaginal membranes. Formulations containing at least one compound or a salt thereof of the disclosure useful for such membranes, may be for example solutions, sprays, suspensions, gels, creams or ointments. Compatible and pharmaceutically acceptable carriers, which may be used in this disclosure, comprise e.g. an aqueous solution, such as saline solutions, oil solutions or ointments. Formulations for ocular use may also contain compatible and pharmaceutically acceptable excipients, such as preservatives, surfactants, stabilizing agents, antibacterial agents, buffering agents and agents such as for example polymers to adjust viscosity, vasoconstrictors, antihistaminic agents or anti-inflammatory agents. Said formulations may be manufactured in different dosage units, suitable for ocular administration. Usually the concentration of active compound in a formulation for use on non-ocular mucous membranes is from 0.01 and 20% by weight.

For example, injectable solutions may contain a vasoconstrictor (e.g. epinephrine or vasopressin); a solution for infusion or regional anesthesia may contain glucose or dextrose, a gel for urogenital topical procedures may contain thickening agents (e.g. hydroxypropylmethylcellulose); a preparation for topical or dermal application may contain penetration promoting agents (e.g. hydroxypolyethoxydodecane, DMSO, DMAC); sprays for topical anesthesia of the mouth and oropharynx may contain saccharin and alcohol, ointments for accessible mucous membranes may contain a lubricant. A compound or a salt thereof of the present disclosure can also be administered together with other membrane stabilizers (local anesthetics), for example to form eutectic mixtures. A compound or a salt thereof of the present disclosure can also be administered together with other therapeutically active compounds, such as capsaicin, Substance-P inhibitors or antagonists, vaso-active compounds, anti-inflammatory agents, etc.

For operations in the fields of oral surgery and dental treatment, in particular, for tooth extraction and the like in the field of dental treatment, compound or a salt thereof of the current disclosure may be used for local injection (agents for local anesthesia).

The composition for local anesthesia of the present disclosure can be provided as a composition for injection in a form of an aqueous solution in which the aforementioned components and optional pharmaceutical additives, which are available for those skilled in the art as additives to be formulated in compositions for topical injections, are dissolved in distilled water for injection.

The composition for local anesthesia of the present disclosure can also be prepared as a pharmaceutical preparation in a dried form such as a lyophilized preparation, and dissolved when used. Generally, the composition is provided for clinical use after being filled in ampoules, vials, cartridges or the like under sterile condition.

In some cases, a compound or a salt thereof of the present disclosure are administered in a therapeutically effective amount. It will be understood, however, that the amount of the compound actually administered will be determined by a physician or clinician, in the light of the relevant circumstances, including the condition to be treated, the chosen route of administration, the compound administered and its relative activity, the age, weight, and response of the individual patient, the severity of the patient's symptoms, and the like.

A compound or a salt thereof of the present disclosure can be prepared by a method well-known to those skilled in the art. However, methods for preparing the composition of the present disclosure are not limited to those described in the examples, and appropriate alterations and modifications can be added to these methods.

### Indications

A compound or a salt thereof of the present disclosure can be used for the induction and/or maintenance of general anesthesia, for example to permit the performance of surgery or other painful procedures; for the initiation and/or maintenance of sedation with patients spontaneously breathing, and for the induction and/or maintenance of sedation for intubated, mechanically ventilated patients.

A compound or a salt thereof of the present disclosure can also be administered in combination with other therapeutic agents, such as, for example, other anesthetics or sedatives, or analgesics (e.g. an opioid). Accordingly, the compositions of the disclosure can optionally further comprise another therapeutic agent, for example, an anesthetic, sedative, or analgesic. Similarly, the therapeutic methods of the disclosure can also optionally comprise administering another therapeutic agent (e.g. an anesthetic, sedative, or analgesic) to the mammal.

Alternatively, a continuous infusion of a compound or a salt thereof of the present disclosure can be used to maintain anesthesia or sedation following induction with another sedative hypnotic agent. Or, in yet another alternative protocol, a bolus dose of a compound or a salt thereof of the current disclosure to induce anesthesia or sedation can be followed by infusion of a different sedative hypnotic agent.

The duration of action of a local anesthetic is proportional to the time during which it is present at effective concentrations in contact with the nerve, or, more precisely, the ion channel(s). The effect of most currently used local anesthetics tends to be short-lived as a result of dissociation from and diffusion away from the intended site of action; therefore, repeated doses must be administered for a prolonged effect. Undesired side effects of local anesthetics are largely a function of systemic concentrations of the drug resulting from such diffusion. These effects include paralysis of cardiac and smooth muscle systems, or undesired stimulation of the CNS. Because of these serious side effects, the quantity of drug administered must be carefully controlled.

Objectives of the present disclosure include methods of inducing anesthesia, inducing sedation, treating central nervous system disorders, peripheral nervous system disorders, psychiatric disorders, ischemia, insomnia, or treating with an anticonvulsant.

A compound or a salt thereof of the present disclosure can be used to induce anesthesia in a patient by administration of a therapeutically effective amount of compound or a salt thereof to a subject. A compound or a salt thereof of the present disclosure can be used to induce anesthesia within at least the two broad categories of anesthesia: general anesthesia and conduction, or local, anesthesia. General anesthesia suppresses central nervous system activity and results in unconsciousness and total lack of sensation. Conduction anesthesia, or regional or local anesthesia, blocks transmission of nerve impulses between a targeted part of the body and the spinal cord, and causes loss of sensation in the targeted body part. A subject treated with a compound or a salt thereof of the current disclosure and under conduction anesthesia may remain fully conscious. A compound or a salt thereof of the current disclosure may also be used within at least two categories of regional anesthesia. A peripheral blockade inhibits sensory perception in a local body part, such as, for example, the numbing of a tooth area for dental work or administering a nerve block to stop sensation from an entire limb. A central blockade administers the anesthetic around the spinal cord, suppressing all sensation below the block. For example, instances of local anesthetics include, but are not limited to, infiltration anesthesia, perisurgical tissue anesthesia, field block anesthesia, peripheral nerve block anesthesia, epidural anesthesia, spinal anesthesia, bier block anesthesia, and combinations thereof.

Sedation is the depression of a subject's awareness to the environment and lowering of general responsiveness to external stimulation. A few levels of sedation include minimal sedation, moderate sedation, deep sedation, and general anesthesia.

Applications that may use a compound or a salt thereof of the current disclosure to induce anesthesia include, but are not limited to, local surgery, regional surgery, arm surgery, hand surgery, face, neck, head, torso, abdominal, lower abdominal pelvic, rectal, lower extremity surgery, minor operations in oral surgery and dental treatment, such as operations which can be completed in several to ten minutes such as tooth extraction in dental treatment. However, applicable operations are not limited to the uses in the oral surgery and dental treatment, and the composition can be used for surgical local anesthesia such as for skin incision. Applications that may use a compound or a salt thereof of the current disclosure to induce infiltration anesthesia include, but are not limited to, subcutaneous infiltration, including IV placement, superficial/shave biopsy, suturing; wound infiltration, including postoperative pain control at incision site; intraarticular injections, including postsurgical pain control, arthritic joint pain control; and infiltrative nerve blocks, including ankle block, scalp block, Bier block, wrist block, and digit block.

Types of surgeries that may need a method of inducing anesthesia with a compound or a salt thereof of the current disclosure includes, but is not limited to, dental surgery, oral surgery, cosmetic surgery, upper body surgery, lower body surgery, abdominal liposuction, adrenalectomy, alveolar cleft surgery, ankle replacement, aortic valve repair surgery, arm biopsy, arm liposuction, arthrocentesis, arthroscopic finger fusion, arthroscopic foot joint replacement, arthroscopic revision hip surgery, arthroscopic total knee replacement, back and neck surgery, back surgery, beating heart pulmonary artery valve replacement, bilateral salpingectomy, bone grafting, breast reconstruction, breast augmentation, brow lift, bunion surgery, buttock lift, calf augmentation, cardiopulmonary bipass, cataract surgery, central venous catheter placement, cerebral aneurysm repair, cervical spine fusion, Cesarean section delivery, cheek augmentation, chest wall resection, chin lift, cleft lip surgery, colectomy, cornea transplant, coronary artery bypass, elbow replacement, endoscopic microdiscectomy, eyelid reconstruction, face lift, face transplant, facial injection, fat grafting, female to male sex reassignment, gastric bypass surgery, hair grafting, heart bypass surgery, heart transplant, heart valve repair surgery, hip replacement surgery, hip surgery, in vitro fertilization, laparoscopic surgeries, laser-assisted in situ keratomileusis (LASIK), liver transplant, lung transplant, male to female sex reassignment, minimally invasive surgeries, neck lift, nerve repair, open surgeries, pacemaker implant, pacemaker surgery, pancreas transplant, prostate removal surgery, quadruple bypass surgery, radial keratotomy, revision surgeries, robotic surgeries, shoulder surgery, skin cancer surgery, stereotactic body radiation therapy, surgical bypass, thumb fusion, thyroid removal surgery, toe fusion, tongue surgery, tooth extraction, ultrasound surgery, vaginoplasty, vasectomy, and wrist replacement surgery.

Without wishing to be bound by theory, a compound that may be used as an anesthetic may be a compound that binds to the cellular membrane, acts on the central nervous system, is able to achieve high concentration within the body, or a combination thereof.

The compositions and methods disclosed herein can be utilized to treat a neurological disease or disorder. In some cases, the neurological disease or disorder is pain. Pain can be acute pain or can be chronic pain. Pain can be nociceptive pain (i.e., pain caused by tissue damage), neuropathic pain or psychogenic pain. In some cases, the pain is caused by or associated with a disease (e.g., cancer, arthritis, diabetes). In other cases, the pain is caused by injury (e.g., sports injury, trauma). Non-limiting examples of pain that are amenable to treatment with the compositions and methods herein include: neuropathic pain including peripheral neuropathy, diabetic neuropathy, post herpetic neuralgia, trigeminal neuralgia, back pain, neuropathy associated with cancer, neuropathy associated with HIV/AIDS, phantom limb pain, carpal tunnel syndrome, central post-stroke pain, pain associated with chronic alcoholism, hypothyroidism, uremia, pain associated with multiple sclerosis, pain associated with spinal cord injury, pain associated with Parkinson's disease, epilepsy, osteoarthritic pain, rheumatoid arthritic pain, visceral pain, and pain associated with vitamin deficiency; and nociceptive pain including pain associated with central nervous system trauma, strains/sprains, and burns; myocardial infarction, acute pancreatitis, post-operative pain, posttraumatic pain, renal colic, pain associated with cancer, pain associated with fibromyalgia, pain associated with carpal tunnel syndrome, and back pain.

Compositions of the current disclosure may also be used in personal care products. Personal care products may include, but are not limited to, shave cream, toothpaste, creams, gels, lotions, ointments, wax, or combinations thereof.

The compositions and methods herein may be utilized to ameliorate a level of pain in a subject. In some cases, a level of pain in a subject is ameliorated by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or 100%. A level of pain in a subject can be assessed by a variety of methods. In some cases, a level of pain is assessed by self-reporting (i.e., a human subject expresses a verbal report of the level of pain he/she is experiencing). In some cases, a level of pain is assessed by behavioral indicators of pain, for example, facial expressions, limb movements, vocalization, restlessness and guarding. These types of assessments may be useful for example when a subject is unable to self-report (e.g., an infant, an unconscious subject, a non-human subject). A level of pain may be assessed after treatment with a composition of the disclosure as compared to the level of pain the subject was experiencing prior to treatment with the composition.

Anesthetic recovery can be categorized in at least four main classes. In stage 1, all functions are normal. In stage 2, the subject may be able to maintain itself in a desired position, or can stand and move about, but may still show some sedation effects. In stage 3, the subject may be conscious and all reflexes present, but may not be able to control its body position. In stage 4, the subject may be semi-conscious or unconscious and have minimal or absent reflexes.

Examples of local anesthetics that are used include, but are not limited to, procaine, amethocaine, cocaine, lidocaine (lignocaine), prilocaine, bupivacaine, levobupivacaine, ropivacaine, mepivacaine, and dibucaine. Examples of general anesthetics that are used include, but are not limited to, inhaled agents such as desflurane, enflurane, halothane, isoflurane, methoxyflurane, nitrous oxide, sevoflurane, and xenon, non-opioid intravenous agents such as amobarbital, methohexital, thiamylal, thiopental, diazepam, lorazepam, midazolam, etomidate, ketamine, and propofol, intravenous opioid analgesic agents, such as Alfentanil, Fentanyl, Remifentanil, Sufentanil, Buprenorphine, Butorphanol, diacetyl morphine (heroin), Hydromorphone, Levorphanol, Meperidine, Methadone, Morphine, Nalbuphine, Oxycodone, Oxymorphone, and Pentazocine, and muscle relaxants, such as Succinylcholine, Decamethonium, Mivacurium, Rapacuronium, Atracurium, Cisatracurium, Rocuronium, Vecuronium, Alcuronium, Doxacurium, Gallamine, Metocurine, Pancuronium, Pipecuronium, and Tubocurarine, etomidate, ketamine, ropivicaine, bupivicaine, and combinations thereof.

Side effects may occur after a subject is dosed with anesthesia. Side effects may include, but are not limited to, nausea, vomiting, sore throat, confusion, muscle aches, itching, chills, shivering, postoperative delirium, cognitive dysfunction, malignant hyperthermia, headache, back pain, difficulty urinating, hematoma, pneumothorax, nerve damage, respiratory depression, hypotension, and any combinations thereof.

A compound or a salt thereof of the present disclosure may be induce fewer side effects in a subject when compared to other commonly used anesthetics.

A compound or a salt thereof of the present disclosure can be used to treat a central nervous system disorder by administration of a therapeutically effective amount of compound to a subject. Disorders of the central nervous system include, but are not limited to, schizophrenia, schizoaffective disorders, schizophreniform disorders, delusional syndromes and other psychotic conditions related and not related to taking psychoactive substances, affective disorder, bipolar disorder, mania, depression, anxiety disorders of various etiology, stress reactions, consciousness disorders, coma, delirium of alcoholic or other etiology, aggression, psychomotor agitation and other conduct disorders, sleep disorders of various etiology, withdrawal syndromes of various etiology, addiction, pain syndromes of various etiology, intoxication with psychoactive substances, cerebral circulatory disorders of various etiology, cerebral atrophy, cerebellar atrophy, senile tremor, essential tremor, psychosomatic disorders of various etiology, conversion disorders, dissociative disorders, urination disorders, autism and other developmental disorders, cognitive disorders of various types, including Alzheimer's disease, Parkinson's disease, attention deficit-hyperactivity disorder, sleep disorders, psychopathological symptoms and neurological disorders in the course of other diseases of the central nervous systems. A compound or a salt thereof of the present disclosure can be used to treat sleep disorders, such as insomnia. A compound or a salt thereof of the present disclosure may be used to treat a tremor, tremors, involuntary muscle contractions, or twitching of muscles or body parts of a subject. The tremor may be, for example, cerebellar tremor, dystonia and dystonic tremors, enhanced physiologic tremor, essential tremor, Holmes' tremor, isolated chin tremor, isolated voice tremor, movement disorders, orthostatic tremor, palatal tremor, parkinsonian tremor, psychogenic tremor, Rubral tremor, task-specific tremors, writer's tremor, or any combination thereof. Tremors may affect the hands, arms, eyes, head, and legs of a subject.

In some embodiments, a compound or a salt thereof of the present disclosure can be used to treat a peripheral nervous system disorder by administration of a therapeutically effective amount of compound to a subject.

Disorders of the peripheral nervous system include, but are not limited to, traumatic nerve damage, Charcot-Marie-Tooth disease, amyotrophic lateral sclerosis, spinobulbar muscular atrophy, spinal muscular atrophy, diabetic neuropathy, uremic neuropathy, peripheral neuropathy, peripheral neurodegenerative disease, peripheral nerve trauma, metabolic neuropathy, diabetic neuropathy, uremic neuropathy, toxic neuropathy, chemotherapy induced neuropathy, retroviral drug-induced neuropathy, motor neuron disease, Guillain-Barre syndrome, chronic inflammatory demyelinating polyneuropathy or CIDP, multifocal motor neuropathies or MMN, neuropathy associated with monoclonal components, neuropathy associated with anti-MAG gammopathy, myasthenia gravis, Lambert-Eaton syndrome, restless leg syndrome, and Stiff Man Syndrome.

In some cases, a compound or a salt thereof of the present disclosure can be used as an anticonvulsant by administration of a therapeutically effective amount of compound to a subject. An anticonvulsant may be used to control and/or prevent seizures or to stop an ongoing series of seizures. In some embodiments, the seizure may be Absence Seizures, Atypical Absence Seizures, Atonic Seizures, Clonic Seizures, Myoclonic Seizures, Tonic Seizures, Tonic-Clonic Seizures, Simple Partial Seizures, Complex Partial Seizures, Secondarily Generalized Seizures, Febrile Seizures, Nonepileptic Seizures, or Refractory Seizures. In some embodiments, a compound or a salt thereof of the disclosure may be used to treat a seizure in a subject, wherein the subject is a human, a dog, a cat, a horse, or a mouse.

In some cases, the methods and compositions of the disclosure are utilized to treat epilepsy. In some embodiments, the nervous system disorder is Angelman syndrome (including, for example, ubiquitin E3 ligase mutation), Benign Rolandic Epilepsy, CDKL5 Disorder, Childhood and Juvenile Absence Epilepsy, Dravet Syndrome, Frontal Lobe Epilepsy, Glut1 Deficiency Syndrome, Hypothalamic Hamartoma, Infantile Spasms/West's Syndrome, Juvenile Myoclonic Epilepsy, Landau-kleffner Syndrome, Lennox-Gastaut Syndrome (LGS), Epilepsy with Myoclonic-Absences, Ohtahara Syndrome, Panayiotopoulos, PCDH19 Epilepsy, Progressive Myoclonic Epilepsies, benign myoclonic epilepsy of infancy, benign neonatal familial convulsions (including, for example, potassium voltage-gated channel subfamily Q (KCNQ) mutation), Rasmussen's Syndrome, Ring Chromosome 20 Syndrome, Reflex Epilepsies, Temporal Lobe Epilepsy, Kohlschutter-Tonz syndrome, Doose syndrome, Myoclonic-astatic Seizures, Myoclonic-atonic seizures, Epileptic encephalopathy, or Neurocutaneous Syndrome.

In some cases, the methods and compositions may be used to treat status epilepticus. A patient may experience a seizure of one, two, three, four, five minutes or more. In some cases, a seizure may last about 1 second, 2 seconds, 3 seconds, 4 seconds, 5 seconds, 10 seconds, 30 seconds, 1 minute, 2 minutes, 3 minutes, 4 minutes, 5 minutes, 10 minutes, or longer.

In some cases, a patient may experience one, two, three, four, five, or more seizures within a given time frame. In some cases, a patient may experience one, two, three, four, five, or more seizures within a five-minute period. A patient may or may not return to normal between seizures.

In some cases, compositions described herein may be used to prevent or control epileptic seizures. Epileptic seizures may be classified as tonic-clonic, tonic, clonic, myoclonic, absence or atonic seizures. The severity of epilepsy may be measured with a known scale, such as, for example, the VA scale, the Chalfont National Hospital scale, the Liverpool scale, the Hague scale, or the Occupational Hazard scale.

In some cases, the compositions and methods herein may prevent or reduce the number of epileptic seizures experienced by a subject by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or 100%.

In some cases, the compositions and methods herein may prevent or reduce the severity of epileptic seizures experienced by a subject by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or 100%.

In some cases, the compositions and methods herein may increase the quality of life (QoL) of a subject by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or 100%.

In some cases, a compound or a salt thereof of the present disclosure can be used to treat psychiatric disorders by administration of a therapeutically effective amount of compound to a subject. Psychiatric disorders may also be known as mental disorders, mental illnesses, or psychological disorders. Non-limiting examples of psychiatric disorders include acute stress disorder, adjustment disorder, adolescent antisocial behavior, alcohol abuse, amphetamine dependence, alcohol dependence, anorexia nervosa, antisocial personality disorder, attention deficit disorder, attention deficit hyperactivity disorder, binge eating disorder, bipolar disorder, bulimia nervosa, caffeine-related disorder, claustrophobia, cocaine dependence, depression, dyslexia, dissociative identity disorder, euphoria, Huntington's disease, major depressive disorder, mean world syndrome, melancholia, minor depressive disorder, mixed episode, Munchausen's syndrome, narcolepsy, nicotine withdrawal, nightmare disorder, obsessive-compulsive disorder (OCD), obsessive-compulsive personality disorder, pain disorder, panic disorder, paranoid personality disorder, Parkinson's disease, perfectionism, physical abuse, posttraumatic stress disorder (PTSD), schizophrenia, seasonal affective disorder, sedative-related disorder, separation anxiety disorder, sleep disorder, and combinations thereof.

In some cases, a compound or a salt thereof of the present disclosure can be used to treat depression by administration of a therapeutically effective amount of compound to a subject. Types of depression, for example, include major depression, persistent depressive disorder, bipolar disorder, seasonal affective disorder, psychotic depression, postpartum depression, premenstrual dysphoric disorder, situational depression, and atypical depression.

In some cases, depression screening measures can provide an indication of the severity of symptoms for a time period. Several rating scales may be used for this purpose, including, but not limited to, the Hamilton depression rating scale, the Montgomery-Asberg depression rating scale, Beck depression inventory, Beck depression inventory PC, Amritsar depression inventory, Beck hopelessness scale, Caroll depression scales, Centre for epidemiological studies depression scale, clinical assessment of depression, depression anxiety stress scales, Edinburgh postnatal depression scale, major depression inventory, patient health questionarie (PHQ-9) screening instrument, Zung self-rating depression scale, and Zung depression inventory.

For any rating scale used, a compound or a salt thereof of the present disclosure can be used to affect a desirable change in a patient. For example, when using the Hamilton depression rating scale, a patient's score may decrease after treatment with a compound or a salt thereof of the current disclosure. The score of the patient may decrease by more than 1 point, 2 points, 3 points, 5 points, 10 points, 15 points, 20 points, 30 points, 40 points, 50 points, or 60 points.

In some cases, a compound or a salt thereof of the present disclosure can be used to treat ischemia by administration of a therapeutically effective amount of compound to a subject. Ischemia is an inadequate blood supply to an organ or part of the body, and can be, for example, myocardial ischemia, cerebral ischemia, small or large intestine ischemia, brain ischemia, limb ischemia, cutaneous ischemia, or combinations thereof.

In some embodiments, a compound or a salt thereof of the present disclosure can be used to treat disorders of the brain, such as, for example, lissencephaly. In some embodiments, the brain disorder may by the Lis-1 mutation or Lis-1 related lissencephaly.

In some cases, a compound or a salt thereof of the present disclosure can be used to treat disorders of the ear, such as auditory neuropathy, by administration of a therapeutically effective amount of compound to a subject. Auditory neuropathy is a type of hearing impairment and can be, for example, auditory neuropathy spectrum disorder (ANSD).

In some cases, a compound or a salt thereof of the present disclosure can be used to treat disorders of the eye by administration of a therapeutically effective amount of compound to a subject. Disorders of the eye can include, but are not limited to, blurred vision, macular degeneration, age-related macular degeneration (AMD or ARMD), retinal degeneration (rhodopsin mutation), eye floaters, eyelid inflammation, eye pain, and glaucoma.

In some cases, a compound or a salt thereof of the present disclosure can be used to treat spasticity by administration of a therapeutically effective amount of compound to a subject. Spasticity in a subject may be a result of other diseases, such as cerebral palsy, multiple sclerosis, traumatic brain injury, stroke, or spinal cord injury. Spasticity may be measured by the amount of muscle rigidity, and measurements may be done according to scales such as the Ashworth Scale, Modified Ashworth Scale, or Bohannon & Smith Scale. The Modified Ashworth Scale consists of scores ranging from 0-4. Treatment with a compound or a salt thereof of the current disclosure may decrease a subject's score by 0 points, 1 point, 2 points, 3 points, 4 points.

In some cases, a compound or a salt thereof of the present disclosure can be used to treat symptoms of itching or pruritis by administration of a therapeutically effective amount of compound to a subject. Itching may be caused by other conditions, including, but not limited to, psychological problems, stress, anxiety, dry skin, sunburns, metabolic and hormone disorders such as liver or kidney diseases, cancers, reactions to drugs, diseases of the blood, allergic reactions, insect stings or bites, infections, infestation by lice or mites, or combinations thereof.

In some cases, a compound or a salt thereof of the current disclosure can be used to treat b-12 deficiency, hyperthyroidism, hyperparathyroidism, hypocalcemia, hyponatremia, kidney disease, liver disease, or Wilson's disease.

In some cases, a compound or a salt thereof of the current disclosure can be used to treat alcohol related disorders, arsenic poisoning, caffeine related disorders, cocaine related disorders, dichlorodiphenyltrichloroethane (ddt), lead, or toluene poisoning, or nicotine related disorders, such as nicotine withdrawal.

In some cases, a compound or a salt thereof of the present disclosure can be used for weight loss. A compound or a salt thereof of the present disclosure may suppress appetite, or cause a patient to lose interest in consuming food. A compound or a salt thereof of the current disclosure may be part of a diet regiment, diet program, or diet supplement.

In some cases, a compound or a salt thereof of the current disclosure can be used to treat asthma or allergies, or symptoms thereof, such as shortness of breath, wheezing, coughing, and inhibition in breathing. A compound may be a bronchodilator, or a substance that dilates bronchi and/or bronchioles. A compound may decrease resistance in the respiratory airway and may increase airflow and oxygen to the lungs.

Action potentials are generated in nerve and muscle cells by ion currents that pass selectively across plasma membranes through transmembrane ion channels. Though not wishing to be bound by theory, a compound or a salt thereof of the present disclosure may act on the cell membrane by adsorption or partitioning into the membrane. The cell membrane may be a lipid bilayer. Physical properties of the membranes may be altered by a compound or a salt thereof of the current disclosure, including, but not limited to, distribution of lipids in the membrane (e.g. into rafts or domains), elastic constants of the membrane, electrostatic potentials of the membrane, fluidity, lateral pressure profiles, stress distributions, melting events, phase transitions or properties, thickness of the membrane, surface tension, or combinations thereof. In some embodiments of the current disclosure, a compound or a salt thereof disclosed herein act on the cell membrane by altering the lateral pressure profile.

In some cases, methods that may be used to observe physical properties of a cell membrance, such as the distribution of lipids or the thickness of a cell membrane, include, but are not limited to, electron microscopy, transmission electron microscopy (TEM), freeze-fracture electron microscopy, x-ray diffraction, x-ray reflectometry, neutron scattering, nuclear magnetic resonance, and combinations thereof.

In some cases, a compound or a salt thereof of the present disclosure may be interfacially-active, have interfacial active properties, surface active properties, or combinations thereof.

Though not wishing to be bound by theory, neurotransmitters in the GABA family may bind to the corresponding receptor and elicit currents through the reception or the neurotransmitters may elicit adsorption onto membranes, modulating currents through the receptor that the neurotransmitter elicited by binding. A compound or a salt thereof of the present disclosure may bind to gamma-aminobutyric acid (GABA), GABA-A receptors, glycine receptors, acetylcholine receptors, serotonin receptors, glutamate receptors, or combinations thereof.

Kits with unit doses of one or more of the compounds described herein, usually in oral or injectable doses, are provided. Such kits may include a container containing the unit dose, an informational package insert describing the use and attendant benefits of the drugs in treating the disease, and optionally an appliance or device for delivery of the composition.

The kit may further comprise any device suitable for administration of the composition. For example, a kit comprising an injectable formulation of pharmaceutical compositions may comprise a needle suitable for subcutaneous administration and an alcohol wipe for sterilization of the injection site.

In some cases, kits may be provided with instructions. The instructions may be provided in the kit or they may be accessed electronically (e.g., on the World Wide Web). The instructions may provide information on how to use the compositions of the present disclosure. The instructions may further provide information on how to use the devices of the present disclosure. The instructions may provide information on how to perform the methods of the disclosure. In some cases, the instructions may provide dosing information. The instructions may provide drug information such as the mechanism of action, the formulation of the drug, adverse risks, contraindications, and the like. In some cases, the kit is purchased by a physician or health care provider for administration at a clinic or hospital. In some cases, the kit is purchased by a laboratory and used for screening candidate compounds.

The computer system 100 illustrated in FIG. 1 may be understood as a logical apparatus that can read instructions from media 111 and/or a network port 105, which can optionally be connected to server 109 having fixed media 112. The system, such as shown in FIG. 1 can include a CPU 101, disk drives 103, optional input devices such as keyboard 115 and/or mouse 116 and optional monitor 107. Data communication can be achieved through the indicated communication medium to a server at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present disclosure can be transmitted over such networks or connections for reception and/or review by a party 122 as illustrated in FIG. 1.

### EXAMPLES

The following examples are given for the purpose of illustrating various embodiments of the disclosure and are not meant to limit the present disclosure in any fashion. The present examples; along with the methods described herein are presently representative of preferred embodiments; are exemplary; and are not intended as limitations on the scope of the disclosure. Changes therein and other uses which are encompassed within the spirit of the disclosure as defined by the scope of the claims will occur to those skilled in the art.

### EXAMPLE 1: Efficacy of Compound of Formula I as an Anesthetic

Tadpoles were placed in a petri dish containing aquarium water. Four to eight tadpoles were placed in each petri dish. The aquarium water contained the specified test compound at the listed concentration. When the tadpoles stopped moving, a plastic rod was used to elicit motion once again by contact between the rod and tadpole. The number of tadpoles that did not move when contacted by the rod was recorded. From this and the number of subjects, the percent of tadpoles in which movement was suppressed were calculated.

The tadpoles that did not move when contacted by a rod was then transferred to a petri dish containing aquarium water that did not have a test compound.

After 1 to 30 minutes, they were again contacted with a rod to ensure the anesthetic effect had worn off.

In some examples, subanesthetic concentrations of ethanol were added into the aquarium water. Tadpoles in which movement was prevented with exposure to a test compound and ethanol but not with exposure to a test compound alone were classified as partial anesthetics.

**Table 2. Tadpole Data.**

| **Compound** | **Concentration (mM)** | **Movement suppressed (%)** | **Number of subjects** |
|---|---|---|---|
| Ethyl Malonic Acid | 16 | 0 | 5 |
| - | 62.5 | 0 | 5 |
| - | 125 | 100 | 5 |
| - | 250 | 100 | 5 |
| S-3-hydroxybutyric acid | 100 | 0 | 6 |
| - | 200 | 0 | 6 |
| - | 250 | 14 | 7 |
| - | 300 | 17 | 12 |
| - | 350 | 50 | 6 |
| - | 400 | 92 | 12 |
| - | 500 | 100 | 5 |
| R-3-hydroxybutyric acid | 100 | 0 | 6 |
| - | 200 | 0 | 6 |
| - | 250 | 0 | 6 |
| - | 300 | 0 | 13 |
| - | 350 | 0 | 5 |
| - | 400 | 46 | 13 |
| - | 500 | 100 | 5 |
| Butyric acid | 62.5 | 100 | 5 |
| - | 125 | 100 | 5 |
| - | 250 | 100 | 5 |
| - | 500 | 100 | 5 |
| Mandelic acid | 250 | 0 | 6 |
| - | 300 | 100 | 6 |
| - | 500 | 100 | 5 |
| Glutamine | 25 | 0 | 6 |
| - | 50 | 100 | 6 |
| - | 100 | 100 | 6 |
| 3,3-Dimethylbutyric acid | 25 | 100 | 6 |
| Alpha-Hydroxyhippuric acid | 5 | 33 | 6 |
| - | 5 + 20 EtOH | 66 | 6 |
| - | 10 | 100 | 6 |
| Phenylpyruvic acid | 62.5 | 100 | 6 |
| - | 125 | 100 | 6 |
| 3-Hydroxy-3-methylbutyric acid | 1.6 | 0 | 5 |
| - | 6.25 | 0 | 5 |
| - | 25 | 0 | 5 |
| - | 100 | 100 | 8 |
| - | 50 + 150 EtOH | 100 | 6 |
| 3-Hydroxy-3-methylbutyric acid | 1.6 | 0 | 5 |
| - | 6.25 | 0 | 5 |
| - | 25 | 0 | 5 |
| - | 100 | 100 | 8 |
| - | 50 + 150 EtOH | 100 | 6 |
| L-valine | 7.8 | 0 | 5 |
| - | 31 | 0 | 5 |
| - | 125 | 0 | 5 |
| - | 500 | 40 | 5 |
| - | 500 + 150 EtOH | 100 | 5 |
| 2-Oxoglutaric acid | 250 + 150 EtOH | 100 | 5 |
| - | 200 + 150 EtOH | 86 | 7 |
| 4-Methylvaleric acid | 20 | 0 | 7 |
| - | 20 + 150 EtOH | 100 | 7 |
| Kynurenic acid | 12.5 | 0 | 5 |
| - | 12.5 + 150 EtOH | 100 | 5 |
| Indole-3-acetic acid | 12.5 | 0 | 5 |
| - | 12.5 + 150 EtOH | 100 | 5 |
| 3-Methylhippuric acid | 3.125 | 0 | 5 |
| - | 3.125 + 150 EtOH | 100 | 5 |
| L-Tartaric acid | 50 | 0 | 5 |
| - | 50 + 150 EtOH | 100 | 5 |
| Proline | 103 | 0 | 5 |
| - | 411 + 150 EtOH | 100 | 5 |
| 3-Methylvaleric acid | 20 | 25 | 4 |
| - | 20 + 150 EtOH | 66 | 6 |
| Fumaric acid | 25 | 40 | 5 |
| - | 25 + 150 EtOH | 80 | 5 |
| Isopropyl malonic acid | 1.6 | 0 | 5 |
| - | 6.25 | 0 | 4 |
| - | 25 | 50 | 4 |
| - | 25 + 150 EtOH | 40 | 5 |
| Choline | 46 + 150 EtOH | 0 | 5 |
| - | 100 + 150 EtOH | 40 | 5 |
| - | 120 + 150 EtOH | 66 | 6 |
| Glutaric acid | 7.8 | 0 | 5 |
| - | 31 | 0 | 5 |
| - | 125 | 0 | 6 |
| - | 125 + 150 EtOH | 60 | 5 |
| Arginine | 46.5 | 0 | 5 |
| - | 192 + 150 EtOH | 83 | 6 |
| Tryptophan | 12.5 | 0 | 5 |
| - | 12.5 + 150 EtOH | 80 | 5 |
| 4-Acetylbutyric acid | 25 | 0 | 5 |
| - | 25 + 150 EtOH | 80 | 5 |
| 4-Methylhippuric acid | 50 | 0 | 5 |
| - | 50 + 150 EtOH | 100 | 5 |
| - | 150 EtOH | 0 | 5 |
| - | 25 + 150 EtOH | 33 | 6 |
| L-Serine | 114 | 0 | 5 |
| - | 229 + 150 EtOH | 60 | 5 |
| - | 300 + 150 EtOH | 100 | 6 |
| Isoleucine | 25 | 0 | 5 |
| - | 100 + 150 EtOH | 60 | 5 |
| Dimethylmalonic acid | 125 | 60 | 5 |
| - | 250 | 60 | 5 |
| Succinic acid | 62.5 | 0 | 6 |
| - | 62.5 + 200 EtOH | 66 | 6 |
| Guanidine | 62.5 | 0 | 6 |
| - | 62.5 + 150 EtOH | 100 | 7 |
| Adipic acid | 50 | 0 | 6 |
| - | 50 + 150 EtOH | 83 | 6 |
| Quinaldic acid | 25 | 16 | 6 |
| - | 25 + 150 EtOH | 60 | 5 |
| Suberic acid | 10 | 50 | 6 |
| L-Asparagine | 100 | 0 | 6 |
| - | 100 + 150 EtOH | 66 | 6 |
| 2-Hydroxybutyric acid | 125 | 0 | 7 |
| - | 125 + 150 EtOH | 86 | 7 |
| 3-Methyl-2-oxovaleric acid | 250 | 33 | 6 |
| Levulinic acid | 100 + 150 EtOH | 50 | 6 |
| Glycine | 250 + 150 EtOH | 100 | 6 |
| Lysine | 250 + 150 EtOH | 50 | 6 |
| Oxaloacetic acid | 125 + 150 EtOH | 100 | 6 |
| Alanine | 62.5 + 150 EtOH | 20 | 5 |
| - | 125 + 150 EtOH | 80 | 5 |
| Methylsuccinic acid | 250 + 150 EtOH | 43 | 7 |
| Glutamate | 50 + 150 EtOH | 100 | 6 |
| Ethanol control | 100 | 0 | 6 |
| - | 150 | 0 | 6 |
| - | 200 | 0 | 6 |
| - | 250 | 33 | 6 |

### EXAMPLE 2: Efficacy of a Compound of Formula I as an Anesthetic in mice

Solutions of varying molarity of a compound of Formula I were prepared.

Butyric acid was dissolved in water to form an 8 M solution. 0.5 mL of the 8 M solution of butyric acid was injected subcutaneously (at the back of the neck) into the mouse. The mouse became anesthetized or lost consciousness after 10-15 minutes, and woke up several hours later. 0.2 mL of the 8 M solution of butyric acid injected intravenously through the mouse tail vein was lethal to the mouse.

### EXAMPLE 3: Efficacy of a Compound of Formula I as an Anesthetic in mice

Solutions of varying molarity of a compound of Formula I were prepared.

3-hydroxybutyric acid was dissolved in water to form 1 M, 1.5 M, and 2 M solutions. 0.2 mL of a 1.5 M solution of 3-hydroxybutyric acid was injected into a tail vein of a CD-1 mouse. The mouse became unconscious or anesthetic for a certain period of time, which was recorded. The mouse eventually woke up and became conscious once again. This was repeated for at least 2 more times on subsequent days. A concentration of 2 M was lethal to the mouse. A concentration of 1 M did not have any observable effect on the mouse.

### EXAMPLE 4: Efficacy of a Compound of Formula I as an Anesthetic in mice

Solutions of varying molarity of a compound of Formula I were prepared.

Ethylmalonic acid was dissolved in water to form 2 M, 3 M, and 4 M solutions. 0.2 mL of a 3 M solution of ethylmalonic acid was injected into a tail vein of a mouse. The mouse became unconscious for a certain period of time, which was recorded. The mouse eventually woke up and became conscious once again. This was repeated for at least 2 more times on subsequent days. A concentration of 4 M was lethal to the mouse. A concentration of 2 M did not have any observable effect on the mouse.

### EXAMPLE 5: Efficacy of a Compound of Formula I as an Anesthetic in mice

Solutions of varying molarity of a compound of Formula I were prepared.

Diethyl ethyl malonate, ethyl (S)-(+)-mandelate, ethyl isovaleric acid, ethyl butyrate, and diethyl ethylphenylmalonate were dissolved in intralipid to form 62.5 mM, 250 mM, and 1 M solutions.

Intralipid alone had no effect on the mice. 62.5 mM solutions of diethyl ethyl malonate, ethyl (S)-(+)-mandelate, ethyl isovaleric acid, ethyl butyrate, and diethyl ethylphenylmalonate injected intravenously caused the animals to be lethargic and those loss coordination. 250 mM and 1M solutions of diethyl ethyl malonate, ethyl (S)-(+)-mandelate, ethyl isovaleric acid, ethyl butyrate, and diethyl ethylphenylmalonate injected intravenously were lethal to the animals.

### EXAMPLE 6: Efficacy of a Compound of Formula I as an Anesthetic in mice

Solutions of varying molarity of a compound of Formula I were prepared.

Ethylmalonic acid was dissolved in water to form a 4 M solution. 0.5 mL of the 4 M solution of ethylmalonic acid was injected subcutaneously into the mouse. The mouse became sedated, anesthetized, or lose consciousness.

### EXAMPLE 7: Efficacy of a Compound of Formula I as an Anticonvulsant in mice

CF-1 mice were used in this study. Number 20 PE tubing to cannulate a mouse tail vein was used. An infusion pump was used to deliver a solution at a rate of 0.34 mL/min. A hemostat was used to prevent backflow in the PE tubing before the experiment stated. To begin the experiment, the hemostat that was clamped to the PE 20 tubing was removed, the infusion of the solution was started into the tail IV of the mouse, and the timer was started.

The study reflects the time in seconds from the start of the infusion to the time of twitch and onset of seizure, or 90 seconds, whichever is sooner. When the seizure occurs, the infusion is turned off.

As a control, metrazol (pentylenetetrazol) in a 0.5% solution (5 mg/mL) in water was prepared. Pentylenetetrazol is used as a control to study seizure phenomena in and to identify compounds that may control seizure susceptibility. Solutions of 8 M butyric acid and 1.5 M 3-hydroxybutyric acid were made.

The time to twitch and time to seizure of the mouse in seconds is recorded in Tables 3-5, below.

**Table 3. Metrazol alone**

| **Entry** | **Metrazol alone Time to Twitch (seconds)** | **Metrazol alone Time to Seizures (seconds)** |
|---|---|---|
| **1** | 31 | 39 |
| **2** | 36 | 49 |
| **3** | 43 | 49 |
| **4** | 34 | 43 |
| **5** | 33 | 43 |
| **6** | 36 | 42 |
| **7** | 37 | 42 |
| **8** | 52 | 59 |
| **9** | 34 | 45 |
| **Avg.** | **37.3** | **45.7** |

**Table 4. Butyric Acid and Metrazol**

| **Entry** | **Butyric Acid and Metrazol Time to Twitch (seconds)** | **Butyric Acid and Metrazol Time to Seizures (seconds)** |
|---|---|---|
| **1** | 53 | 57 |
| **2** | 35 | 38 |
| **3** | 35 | 65 |
| **4** | 36 | 50 |
| **5** | 38 | 51 |
| **6** | 42 | 50 |
| **7** | 44 | 47 |
| **8** | 50 | 57 |
| **9** | 34 | 42 |
| **10** | 53 | 66 |
| **11** | 34 | 41 |
| **12** | 48 | 57 |
| **13** | 36 | 44 |
| **Avg.** | **41.4** | **51.2** |

**Table 5. 3-Hydroxybutyric Acid and Metrazol**

| **Entry** | **3-Hydroxybutyric Acid and Metrazol Time to Twitch (seconds)** | **3-Hydroxybutyric Acid and Metrazol Time to Seizures (seconds)** |
|---|---|---|
| **1** | 45 | 65 |
| **2** | 41 | 48 |
| **3** | 56 | 61 |
| **4** | 54 | 74 |
| **5** | 49 | 53 |
| **6** | 47 | 53 |
| **7** | 38 | 48 |
| **8** | 50 | 53 |
| **Avg.** | **47.5** | **56.9** |

The average time to twitch was increased when a mouse was also given either butyric acid or 3-hydroxybutyric acid. The average time to twitch for a mouse when dosed only with metrazol was 37.3 seconds. The average time increased when the mouse was also dosed with butyric acid or 3-hydroxybutyric acid to give an average time of 41.4 and 47.5 seconds, respectively.

The average time to seizure was increased when a mouse was also given either butyric acid or 3-hydroxybutyric acid. The average time to seizure for a mouse when dosed only with metrazol was 45.7 seconds. The average time increased when the mouse was also dosed with butyric acid or 3-hydroxybutyric acid to give an average time of 51.2 and 56.9 seconds, respectively.

### EXAMPLE 8: Acute Intravenous Toxicity in Mice

Male mice, weighing 20 grams to 22 grams, are used after a stabilization period of at least ten days at the testing facility and at least one hour in the laboratory. Food but not water is withheld from all animals for 16 hours before the test. The animals are again given free access to food starting two hours after the drug administration. All animals are observed daily for 7 days post dosing.

### EXAMPLE 9: Efficacy of Compound of Formula I as a Topical Anesthetic

Aliquots (0.25 ml) of solutions with varying amounts of a compound or salt disclosed herein are applied into the conjunctival sac of conscious rabbits (either sex; 2-4 kg) and the eye-lids are kept closed for approximately 20 sec. The corneal reflex is checked before application of the test solution and every 5 min thereafter. To test the corneal reflex, the cornea is touched six times with a stalked elastic bristle. The duration of anesthesia is calculated as the period from the time-point when the animal does not feel any of the six touches by the bristle to the time point when the animal again reacts to three of the six touches. To verify the reversibility of the topical anesthetic effect, the testing continues until the animal reacted to all six touches of the bristle for at least 15 minutes.

### EXAMPLE 10: Efficacy of Compound of Formula I as a Dermal Topical Anesthetic

Approximately 18-24 hours before each experiment, the skin on the back of male guinea pigs is shaved and depilated with a commercially available hair remover. The anesthetic action of each agent following dermal application is determined using a "pin-prick" method. Before and at various intervals after treatment with a compound disclosed herein, the areas of the skin are tested for the presence or absence of a skin twitch in response to six standardized dermal probings with a pointed metal "algesimeter" at a predetermined maximum load of 10 grams. The average number of probings not producing a skin twitch response is designated as the "anesthetic score". In this system six responses to six stimuli represents "no anesthetic activity" and no response to six stimuli represents a "maximal anesthetic activity". In experiments on dermal anesthetic activity, a single area of skin 1 inch square is marked off on the back of each animal. This area is covered by a 1 inch square, 16 layer thick gauze pad onto which was deposited 0.45 ml of a 10% solution of the test agent in water with DMSO. The entire area is then covered by wrapping an elastic bandage around the trunk of the animal. After a predetermined duration of treatment, the coverings are removed and the skin is assessed for the presence of anesthesia as described above. Dermal anesthesia is assessed at ten minute intervals to measure onset time and duration of dermal anesthetic activity; comparisons are made with reference compounds and vehicle.

### EXAMPLE 11: Inducing Anesthesia in a Patient Undergoing Oral Surgery with a Compound of Formula I

Before undergoing surgery, patients are induced with a compound of Formula I or a salt thereof via intravenous injection (5 mg/kg dosing). All patients are allowed to breathe spontaneously with manual assistance when needed throughout the procedure. No further muscle relaxant or opioid analgesic is used. Anesthesia is maintained by inhaling 60% nitrous oxide in oxygen via a circle anesthesia breathing system.

Patient characteristics, neurobehavioral and sympathetic responses (movement of body or arm, eye opening, tearing, sweating, and catecholamine levels) are monitored by the anesthesiology team and, in addition, by an extra observer. Since patients were not paralyzed, any of the above signs will be noticed if there is an inadequate level of anesthesia at any time during surgery.

### EXAMPLE 12: Treatment of Pain Relief for a Patient after Surgery with a Compound of Formula I

A patient that has undergone surgery (1 day - 4 weeks) that does not respond to conventional treatment is treated with a compound of Formula I or a salt thereof. Dosage regimens depend on the level of pain the patient experiences. Patients are dosed with an amount of compound of Formula I or a salt thereof, and then evaluated 1 hour, 4 hours, and 8 hours after treatment. After evaluation, the dosage is increased, decreased, or kept the same depending on the change in the symptoms of pain. The treatment is maintined for as long as necessary to affect a stable resolution of the symptoms.

### EXAMPLE 13: Treatment of Pain Relief for a Patient with Chronic Pain with a Compound of Formula I

Patients that are suffering from chronic pain have their pain levels assessed and evaluated. Patients are prescribed 10 mg/day of a compound of Formula I or a salt thereof, and then evaluated again after two weeks to determine if symptoms have improved. After evaluation, the dosage is increased, decreased, or kept the same depending on the change in the level of pain. The treatment is maintained for as long as necessary to affect a stable resolution of the symptoms of chronic pain.

### EXAMPLE 14: Efficacy of Compound of Formula I in Treating Cerebrovascular Disorders such as Cerebral Ischemia

Seven-week-old male rats are housed in polymethylpentene cages in an animal room controlled for room temperature, relative humidity, ventilation rate, and light-dark cycle for at least 6 days. The animals are allowed free access to pellet diet and water from water bottles. Animals judged to be in good health are used.

Rats are anesthetized by inhalation of a gas mixture of 2% isoflurane and N₂O:O₂ (=7:3), stabilized in the supine position, and maintained in the anesthetized state by inhalation of the above gas mixture. The animals are monitored for rectal temperature using a temperature probe during the period of the surgical operation. When a fall in body temperature is observed, an incandescent lamp is used to maintain the temperature at around 37° C. The right common carotid artery, external carotid artery, and internal carotid artery are exposed for occluding the middle cerebral artery (MCA). The right common carotid artery and the external carotid artery are ligatured using sutures (5-0), and a 19 mm-long segment of No. 4-0 nylon suture which are precoated with silicone was inserted into the MCA through the bifurcation of the external and internal carotid arteries to occlude the MCA. At 2 hours after the MCA occlusion, the suture was removed and the blood flow in the MCA was restored.

A compound or a salt thereof of the current disclosure is dissolved in a vehicle and administered intravenously to the animals at a volume of 2 mL/kg immediately after the MCA occlusion-reperfusion and 30 minutes after the MCA occlusion-reperfusion. The control group receives an equal volume of the vehicle in the same manner.

At 24 hours after MCA occlusion, the animals are decapitated and the brains are immediately isolated. Sequential brain sections with a thickness of 2 mm are prepared. The brain tissue sections are positioned to include the coronal plane at 4 mm anterior to the bregma, at 2 mm anterior to the bregma, at the bregma, at 2 mm posterior to the bregma, at 4 mm posterior to the bregma, and at 6 mm posterior to the bregma. The brain sections are stained in 1% TTC solution and photographed, and the infarct area was measured. Based on these results, the infarct volume (4 mm anterior to the bregma—6 mm posterior to the bregma) is calculated. The decrease in volume after ischemia in a dose-dependent manner indicates that a compound or a salt thereof of the current disclosure is useful for the treatment of cerebrovascular disorders such as cerebral ischemia.

### EXAMPLE 15: Efficacy of Compound of Formula I in Treating Central Nervous System Disorders, such as Alzheimer's Disease

Alzheimer's disease model animals are prepared by bilateral ibotenic acid lesions of basal ganglia in rats. Briefly, rats are anesthetized with pentobarbital sodium and placed in a small animal stereotaxic apparatus. Bilateral infusions of 5 µg/0.5 µL of ibotenic acid into the basal ganglia are made at a rate of 0.1 µL/min via a syringe pump and a stainless steel cannula. Stereotaxic coordinates are as follows: -0.8 mm posterior from bregma, 2.6 mm lateral (both sides) from midline, and 7.4 mm depth from the bone surface. Animals in sham group receive only anesthesia. Animals are then housed with free access to food and water for the rest of the study.

A compound or a salt thereof of the current disclosure is administered orally for 14 days after surgery to the model animals. A control group receives the same amount of the vehicle.

Morris water maze test is performed to evaluate the effect of a compound or a salt thereof of the current disclosure. The water maze is a circular pool. During testing in the water maze, a platform, 12 cm in diameter, is located 2 cm below the water in one of four locations (zone 4) in the pool, approximately 38 cm from the sidewall. A light bulb is placed around the pool as a cue external to the maze. The animals receive 2 trials per day from 10 days after the initiation of the administration with a compound or a salt thereof of the current disclosure or the vehicle. The rats are trained to locate the hidden escape platform, which remain in a fixed location throughout testing. Trials last a maximum of 90 sec. The latency to find the submerged platform is recorded and used as a measure of acquisition of the task. The animals are tested in this way for 4 days (total 8 trials), and then they receive a probe trial on the 5th day. For the probe trial, the platform is removed from the pool and then the animal is released from the quadrant opposite to where the platform would have been located. The length of the trial was 90 sec, after which the rat was removed from the pool. The time the rat spends searching for the platform in the training quadrant (zone 4): i.e., the previous location of the platform, is recorded and used as an index of memory.

### EXAMPLE 16: Treatment of a Patient that has been Diagnosed with Alzheimer's Disease

Patients that are diagnosed clinically with Alzheimer's disease are evaluated for common symptoms such as memory loss and confusion.

Patients are prescribed 10 mg/day of a compound of Formula I or a salt thereof, and then evaluated again after two weeks to determine if symptoms have worsened. After evaluation, the dosage is increased, decreased, or kept the same depending on the change in the symptoms of inattention and hyperactivity. The treatment is maintained for as long as necessary to affect a stable or desired level of the symptoms of Alzheimer's disease.

### EXAMPLE 17: Treatment of a Patient that has been Diagnosed with Disorders of the Peripheral Nervous System

A compound of Formula I or a salt thereof or a control is given to patients that have been previously diagnosed with Guillain-Barre syndrome. After administration of a compound of Formula I or a salt thereof, motor strength of the patient is rated on a traditional 0-5 scale:
i. absent motor strength
ii. trace motor strength
iii. can move the specified joint but only with gravity eliminated
iv. can move the joint against gravity but not against any opposing force
v. can move the joint against opposing force but the strength is not normal for the person or symmetrical
vi. normal motor strength

This scale is employed to measure the following motor strength for each of these joint motions on both the right and the left sides: hip flexion, hip adduction, hip abduction, knee flexion, knee extension, ankle dorsi-flexion, ankle plantar flexion, shoulder abduction, elbow extension, elbow flexion, wrist flexion, and wrist extension.

Hand grip strength was measured on a hand dynamometer that had been calibrated. Each patient is given three trials separated by thirty second rest periods and the strongest of the three measurements is recorded for each hand.

In addition, serum laboratories are drawn at the beginning of the study and every week of the study. The serum laboratories include glucose, blood urea nitrogen, creatinine, uric acid, calcium, total protein, albumin, phosphate, total bilirubin, cholesterol, LDH, SGOT/AST, alkaline phosphatase, hematocrit, hemoglobin, red blood cell count, platelet count, and white blood cell count with differential.

The motor strength of the two groups, administration with a compound of Formula I or control, are compared.

### EXAMPLE 18: Anticonvulsant and Antiepileptic Effects of a Compound of Formula I

Adult male Sprague-Dawley rats are anesthetized with ketamine (80 mg/kg intramuscularly) and xylazine (10 mg/kg intramuscularly), and are stereotactically implanted with an insulated stainless steel bipolar electrode for stimulation and recording. The electrode is implanted in the perforant path (8.1 mm posterior, 4.4 mm lateral, 3.5 mm ventral with respect to bregma), and is fixed to the skull with acrylic. After a two-week recovery period following electrode placement, unrestrained, awake, implanted rats receive twice-daily kindling stimulation (5 days per week) with a one-second train of 62-Hertz (Hz) biphasic constant current 1.0-millisecond (ms) square wave pulses to induce kindled seizures. The electroencephalogram is recorded from the bipolar electrode, which is switched After a two-week recovery period following electrode placement, unrestrained, awake, implanted rats receive twice-daily kindling stimulation (5 days per week) with a one-second train of 62-Hertz (Hz) biphasic constant current 1.0-millisecond (ms) square wave pulses to induce kindled seizures. the stimulator for the delivery of kindling stimulation. On the first day of stimulation, each rat receives a stimulus train of 500 microAmperes (µA). If a seizure is evoked, this intensity is used in subsequent stimulations. If no seizure is evoked, the stimulation intensity is increased in a sequence of 500, 700, 900, 1000, 1100, 1200, 1300 and 1400 µA until a seizure is evoked. The intensity that initially evoked seizure is used for subsequent stimulations.

The anticonvulsant and antiepileptic effects of a compound of Formula I or a salt thereof are determined by comparing the rats that require a larger number of seizures to reach more severe classes of seizures than saline treated controls.

### EXAMPLE 19: Efficacy of Compound of Formula I in Preventing Seizure Spread

A Maximal Electroshock Seizure (MES) test is used as a model for behavioral and electrographic seizures that are consistent with those observed in humans. In the MES test, an animal receives an electrical stimulus, 0.2 seconds in duration, via corneal electrodes primed with an electrolyte solution containing an anesthetic agent. The 0.2 second stimulation is generated with 150 mA in rats and 50 mA in mice at 60 Hz. Rats, weighing from 105 g and 130 g, and mice, weighing from 18 g and 25.5 g, receive an electrical stimulus 15 minutes, 30 minutes, 1 hour, 2 hours, and 4 hours after administration of the test compound. In rats, the compound is administered orally, while mice receive the agent via intraperitoneal injection. The test endpoint, electrogenic seizure, is manifested as hindlimb tonic extension. Inhibition of hindlimb tonic extension indicates that the test compound is able to inhibit MES-induced seizure spread and therefore has antiseizure activity.

### Example 20: Efficacy of Compound of Formula I in Preventing and/or Treating Epilepsy

Mice and littermate controls are treated with a compound of Formula I or vehicle starting at postnatal day 14 (early treatment) or six weeks of age (late treatment), corresponding to times before and after onset of neurological abnormalities. Mice are monitored for seizures by serial video-EEG and for long-term survival. Brains are examined histologically for astrogliosis and neuronal organization. Expression of phospho-S6 and other molecular markers correlating with epileptogenesis are measured by Western blotting.

### Example 21: Efficacy of Compound of Formula I as an Anticonvulsant with a Maximal Electroshock Test (MES)

CF-1 male albino mice (25-35 g) were randomly selected into control and test groups, with the animals dosed with vehicle or test compound, at varying time-points and concentrations, respectively. On the study date, the mice are dosed by intraperitoneal injection with vehicle (30% polyoxyethylated 12-hydroxystearic acid) or test compound (50-250 mg/kg). Seizures were induced by trans-corneal electric shock using a 60-Hz alternating current, 50 mA, delivered for 0.2 sec. The mice in the test groups are subjected to electrical stimulus at time intervals from 15 minutes and 4 hours following administration of test compound. The shock resulted in an immediate full body tonic extension. The test was complete when the entire course of the convulsion has been observed (typically, less than 1 minute after electrical stimulation). The ED₅₀ value of the test compounds is calculated, and is the dose required to block the hind limb tonic-extensor component of the MES-induced seizure in 50% of the rodents tested.

### EXAMPLE 22: Efficacy of Compound of Formula I on Social Behavior

The low level of sociability in mice of the BALB/cByJ (hereafter BALB/c) inbred strain is considered to be relevant to autism. This example is used to examine the effects of a compound of Formula I or a salt thereof on the social behavior in BALB/c mice compared control mice. The social investigatory behavior of mice is measured by the amount of time before the "test" mouse engages in social contact with the control, "stimulus" mouse.

All male mice are housed in temperature-controlled rooms. Food and water remained freely available. Individual housing was provided firstly to permit resident male mice an opportunity to scent mark and habituate to their home cage and, secondly to heighten the motivation of "test" mice for social interaction. All the animals were allowed to acclimate to the test room (natural lighting) for at least 1 h prior to testing which was conducted from 9 a.m. and 3 p.m.

Two sets of "test" mice are treated, one with a compound of Formula I or a salt thereof, and the other with saline solution, as a control, at doses of 100, 200 and 300 mg/kg once daily for 5 consecutive days. The last dose was administered 60 min before testing.

The BALB/c mouse is then placed into the mouse cage with a "stimulus" mouse. For each contact, an observer, sitting about 1 m from the cage; recorded the latency to the first contact (or approach) (in sec) towards the "stimulus" mouse and the time (in sec) that the "test" mouse spent in social investigatory behavior. Nosing, sniffing, pawing, grooming and close following of the "stimulus" mouse by the young adult mouse were considered to be signs of social investigation. Aggressive behaviors, such as biting and mounting, are excluded from measurement. The time to first contact or approach is compared between the two sets of "test" mice to determine the effectiveness of a compound of Formula I or a salt thereof for the treatment of autism.

### Example 23: Treatment of a Patient that has been Diagnosed with Inattention, Hyperactivity, or Schizophrenia

Patients with symptoms such as delusions, hallucinations, and disorganized or altered speech are evaluated with conventional testing used by mental health professionals to evaluate schizophrenia.

Patients are prescribed 10 mg/day of a compound of Formula I or a salt thereof, and then evaluated again after two weeks to determine if symptoms have improved. After evaluation, the dosage is increased, decreased, or kept the same depending on the change in the symptoms of schizophrenia. The treatment is maintained for as long as necessary to affect a stable resolution of the symptoms of schizophrenia.

### Example 24: Treatment of a Patient that has been Diagnosed with Depression

Patients with clinical symptoms of depression are evaluated with conventional testing used by mental health professionals to evaluate depression.

Patients are prescribed 10 mg/day of a compound of Formula I or a salt thereof, and then evaluated again after two weeks to determine if symptoms have improved. After evaluation, the dosage is increased, decreased, or kept the same depending on the change in the symptoms of depression. The treatment is maintained for as long as necessary to affect a stable resolution of the symptoms of depression. The severity and amount of clinical symptoms of depression is decreased in the patient.

### EXAMPLE 25: Treatment of a Patient that has been Diagnosed with Psychomotor Retardation

Patients with clinical symptoms of psychomotor retardation such as slowing of coordination, speech, and impaired articulation are evaluated with conventional testing used by mental health professionals to evaluate psychomotor retardation.

Patients are prescribed 10 mg/day of a compound of Formula I or a salt thereof, and then evaluated again after two weeks to determine if symptoms have improved. After evaluation, the dosage is increased, decreased, or kept the same depending on the change in the symptoms of psychomotor retardation. The treatment is maintained for as long as necessary to affect a stable resolution of the symptoms of psychomotor retardation.

### EXAMPLE 26: Treatment of Inattention and Hyperactivity in a Patient that has been Diagnosed with Parkinson's Disease

Patients that are diagnosed clinically with Parkinson's disease are evaluated for inattention and hyperactivity.

Patients are prescribed 10 mg/day of a compound of Formula I or a salt thereof, and then evaluated again after two weeks to determine if symptoms have improved. After evaluation, the dosage is increased, decreased, or kept the same depending on the change in the symptoms of inattention and hyperactivity. The treatment is maintained for as long as necessary to affect a stable resolution of the symptoms of inattention and hyperactivity.

### EXAMPLE 27: Treatment of a Patient that has been Diagnosed with an Anxiety Disorder

Patients with clinical symptoms of anxiety disorders such as slowing of fear, specific phobias, panic attacks, are evaluated with conventional testing used by mental health professionals to evaluate the type and degree of the anxiety disorder.

Patients are prescribed 10 mg/day of a compound of Formula I or a salt thereof, and then evaluated again after two weeks to determine if symptoms have improved. After evaluation, the dosage is increased, decreased, or kept the same depending on the change in the symptoms of anxiety. The treatment is maintained for as long as necessary to affect a stable resolution of the symptoms of the anxiety disorder.

### EXAMPLE 28: Efficacy of a Compound of Formula I for Treatment of Attention Disorders

The timing/peak procedure test is an operant test in which mice are trained to respond to a food reward at a fixed time interval of 30 sec. Mice learn to increase their responding around the 30 sec time period. This test assesses impulsivity, attention and timing perception. A high peak of responding at the 30 sec interval and narrow spread are signatures of the animal's improved attention and time perception.

Mice are placed in a chamber and trained to lever press for food. After training, animals learn to respond after a fixed interval of 30 sec has elapsed as only this fixed interval response produces a reward. With reinforcement delivery the lever is retracted and an inter-trial-interval commences. Once the animal was trained on the reinforced trials, "peak trials" or unreinforced trials are introduced and intermixed with reinforced trials. During these empty trials, no responses were reinforced and the trial lasts for 120 sec (i.e., the lever is extended but reinforcement is not presented). After the 120 sec elapses, the trial terminated, the lever was retracted and the inter-trial-interval started as before. The animals therefore learned that if after a period of 30 sec, no food has been presented there will be no reinforcement delivery until the lever is retracted and the trial starts again.

To perform well on this task, animals need to learn an association between a response (lever pressing) and the delivery of reinforcement (condensed milk), they need to perceive and remember time, they need to act on the remembered time by responding or by inhibiting a response and finally they need to compare the elapsed time during a trial with their memory for the time for reinforcement. Mice are dosed with a compound of Formula I or a salt thereof, and the response times are recorded and compared to control mice.

### EXAMPLE 29: Treatment of a Patient that has been Diagnosed with Spasticity

A compound of Formula I or a salt thereof or a control is given to patients that have been previously diagnosed with muscle spasticity. After administration of a compound of Formula I or a salt thereof, muscle contraction of the patient is rated on a traditional 0-4 scale:
0 ― No increase in tone
1 ― Slight increase in muscle tone, manifested by a catch and release or minimal resistance at the end of the ROM when the affected part(s) is moved in flexion or extension 1+ - Slight increase in muscle tone, manifested by a catch, followed by minimal resistance throughout the remainder (less than half) of the ROM
2 - More marked increase in muscle tone through most of the ROM, but affected part(s) easily moved
3 - Considerable increase in muscle tone, passive movement difficult
4 - Affected part(s) rigid in flexion or extension
The muscle contraction of the two groups of patients, administration with a compound of Formula I or a salt thereof or control, are compared.

### EXAMPLE 30: Treatment of a Patient that has Symptoms of Itching (Pruritus)

A compound of Formula I or a salt thereof or a control is given to patients that show symptoms of itching. After administration of a compound of Formula I or a salt thereof, prutitis of the patient is rated on a traditional 1-5 scale:
1 - Pruritus without the need to scratch
2 - Pruritus with the need to scratch but without excoriation
3 - Pruritus unrelieved by scratching but without excoriation
4 - Pruritus accompanied by excoriation
5 - Totally restless

The prutitis levels of the two groups of patients, administration with a compound of Formula I or a salt thereof or control, are compared.

### EXAMPLE 31: Liquid formulation

A compound of Formula I or a salt thereof may be formulated as a liquid for intravenous administration with the composition listed in Table 6.

**Table 6. Liquid formulation**

| Ingredient | Quantity |
|---|---|
| Sodium Chloride | 0.9 g/100 mL |
| Methyl paraben | 1 mg/mL |
| Compound of Formula I | 0.5 g/100 mL |
| Water | Up to 100 mL |

### EXAMPLE 32: Paste formulation

A compound of Formula I or a salt thereof may be formulated as a paste for topical administration with the composition listed in Table 7.

**Table 7. Paste formulation**

| Ingredient | Quantity (%) |
|---|---|
| Compound of Formula I | 1 |
| Zinc oxide | 25 |
| Starch | 25 |
| Calamine | 5 |
| White petroleum | Up to 100 |

### EXAMPLE 33: Ointment formulation

A compound of Formula I or a salt thereof may be formulated as an ointment for topical administration with the composition listed in Table 8.

**Table 8. Ointment formulation**

| Ingredient | Quantity (%) |
|---|---|
| Compound of Formula I | 10 |
| White wax | 5 |
| White petroleum | Up to 100 |

### EXAMPLE 34: Cream formulation

A compound of Formula I or a salt thereof may be formulated as a cream for topical administration with the composition listed in Table 9.

**Table 9. Cream formulation**

| Ingredient | Quantity (%) |
|---|---|
| Compound of Formula I | 0.5 |
| White wax | 20 |
| Almond oil | 55 |
| Rose water | 2 |
| Rose oil | 0.02 |
| Water | Up to 100 |

### EXAMPLE 35: Gel formulation

A compound of Formula I or a salt thereof may be formulated as a gel for topical administration with the composition listed in Table 10.

### Table 10. Gel formulation

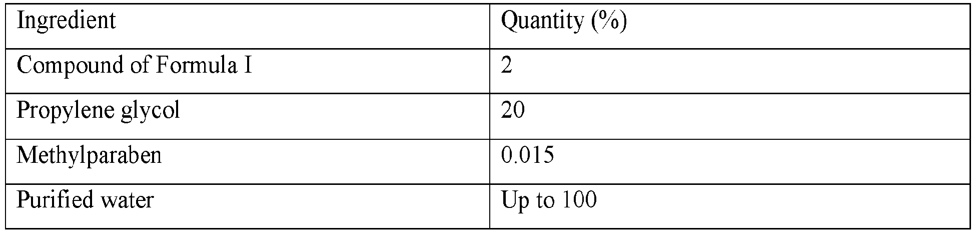

### EXAMPLE 36: Administration of a Compound of Formula (I) via a Patch

A compound of Formula I or a salt thereof is administered to a subject via a transdermal patch. The transdermal patch consists of a compound of Formula I on porous material such as gauze or sponge and a backing layer that holds the porous material. The patch can alternatively consist of microneedles. The backing layer is attached to the skin of a subject so that the porous material is in contact with the skin of a subject.

### EXAMPLE 37: Measurement of Cell Membrane Physical Properties after Dosage with a Compound of Formula (I)

A compound of formula (I) is administered to a subject. Physical properties change after dosage, and various measurements are used to quantify the changes in cell membrane structure. A small sample of cells are harvested from the subject, and the sample is analyzed via transmission electron microscopy.

### EXAMPLE 38: Synthesis of a Compound of Formula I

A compound of Formula I or a salt thereof is synthesized according to Scheme I:

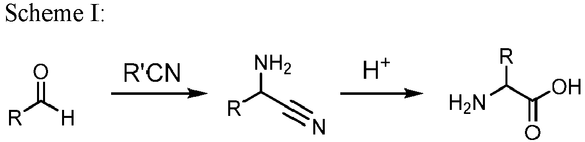

The variable R in Scheme I is selected from substituents known to one skilled in the art. Non-limiting examples of substituents include independently alkyl, alkenyl, alkynyl, alkoxy, acyl, acyloxy, carboxylic acid, ester, amine, amide, carbonate, carbamate, nitro, thioether, thioester, cycloalkyl, heteroalkyl, aryl, and heteroaryl, any of which is substituted or unsubstituted, halogen, hydroxyl, sulfhydryl, nitro, nitroso, cyano, azido, and H.

An aldehyde is condensed with a cyanide to form a nitrile. The nitrile is then hydrolyzed to from the substituted amino acid.

### EXAMPLE 39: Esterification of a Compound of Formula I

A compound of Formula I or a salt thereof is esterified according to Scheme II:

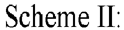

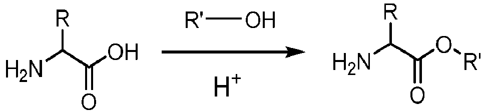

The variable R' in Scheme II is selected from substituents known to one skilled in the art. Non-limiting examples of substituents include independently alkyl, alkenyl, alkynyl, carboxylic acid, ester, amide, carbonate, carbamate, thioester, cycloalkyl, heteroalkyl, aryl, and heteroaryl, any of which is substituted or unsubstituted, and H.

An acid is heated in the presence of an alcohol and an acid, with or without solvent. Reaction times vary on substrates, and can be from 1 minute to 100 hours. Reaction temperatures vary on substrates, and can be from 0 °C to 200 °C.

### EXAMPLE 40: Resolution of a Compound of Formula I

A compound of Formula I or a salt thereof is resolved according to Scheme III:

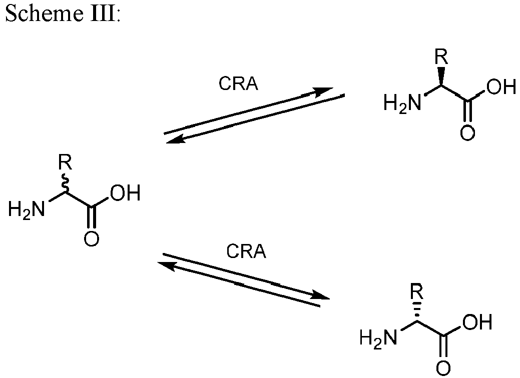

A chiral resolving agent (CRA), or chiral auxiliary, is used to separate a racemic mixture into the enantiomers. Chiral resolving agents are selected from compounds known to one skilled in the art. Non-limiting examples of chiral resolving agents include chiral oxazolidinones and chiral sulfoxides.

A compound of formula I is resolved using a chiral silica gel column.

### EXAMPLE 41: Formation of a Salt of a Compound of Formula I

A salt of a compound of formula I is made according to methods known to those skilled in the art. A salt is formed according to Scheme IV:

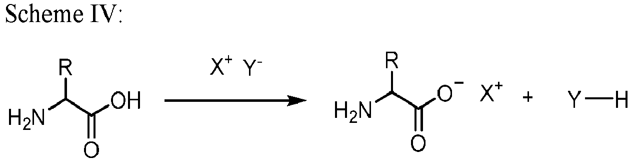

The present invention also relates to the following embodiments:
1. A method comprising administering to a subject in need thereof a therapeutically-effective amount of a compound of Formula (I): 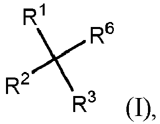 or a salt thereof, wherein:
   R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
   X is O, S, or N;
   Y is O, S, or N;
   R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
   R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
   R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
   A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
   R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
   R⁸ is independently selected at each occurrence from hydrogen and C₁₋₂₀ alkyl; and wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof.
2. The method of embodiment 1, wherein said compound is represented by Formula (I-A): 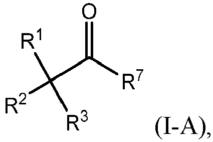 or a salt thereof.
3. A method comprising administering to a subject in need thereof a therapeutically-effective amount of a compound or a salt thereof listed in Table 1 or Table 2, wherein the method is selected from inducing sedation, sedating, treating a central nervous system disorder, treating a peripheral nervous system disorder, treating a convulsing disorder, treating a psychiatric disorder, treating ischemia, treating pain, treating spasticity, treating itching, and any combination thereof.
4. The method of embodiment 1, wherein said compound or a salt thereof is a racemic mixture.
5. The method of embodiment 1, wherein said compound or a salt thereof has an enantiomeric excess of greater than 80%.
6. The method of embodiment 1, wherein said compound or a salt thereof has a diastereomeric excess of greater than 80%.
7. The method of embodiment 1, wherein said compound or a salt thereof has an alkene group, and wherein said alkene group has a cis geometry.
8. The method of embodiment 1, wherein said compound or a salt thereof has an alkene group, and wherein said alkene group has a trans geometry.
9. The method of embodiment 1, wherein said compound or a salt thereof is administered in a formulation.
10. The method of embodiment 1, wherein said administering of a compound or a salt thereof alters distribution of lipids in a cell membrane.
11. The method of embodiment 1, wherein said administering of a compound or a salt thereof alters cell membrane thickness.
12. The method of embodiment 1, wherein said compound or salt thereof is selected from the group consisting of: (S)-3-hydroxybutanoic acid, (R)-3-hydroxybutanoic acid, 3,3-dimethylbutanoic acid, 2-benzamido-2-hydroxyacetic acid, butyric acid, 2-ethylmalonic acid, glutamine, and a salt of any one thereof.
13. The method of embodiment 1, wherein said compound or salt thereof is selected from the group consisting of: butyric acid, 3-hydroxybutyric acid, ethylmalonic acid, diethyl ethyl malonate, ethyl (S)-(+)-mandelate, ethyl isovaleric acid, ethyl butyrate, diethyl ethylphenylmalonate, and a salt of any one thereof.
14. The method of embodiment 1, wherein said compound or salt thereof is selected from the group consisting of: butyric acid, 3-hydroxybutyric acid, ethylmalonic acid, diethyl ethyl malonate, and a salt of any one thereof.
15. The method of embodiment 1, wherein said compound or salt thereof is butyric acid.
16. The method of embodiment 1, wherein said compound or salt thereof is 3-hydroxybutyric acid.
17. A pharmaceutical composition comprising a compound of Formula (I): 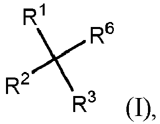 or a salt thereof, wherein:
   R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
   X is O, S, or N;
   Y is O, S, or N;
   R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
   R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
   R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
   A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
   R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S;
   R⁸ is independently selected at each occurrence from hydrogen and C₁₋₂₀ alkyl; and a pharmaceutically-acceptable excipient.
18. The pharmaceutical composition of embodiment 17, wherein said compound is represented by Formula (I-A): 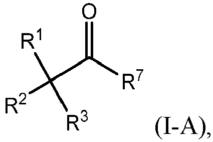 or a salt thereof.
19. A pharmaceutical composition comprising a therapeutically-effective amount of a compound or a salt thereof listed in Table 1 or Table 2, and a pharmaceutically-acceptable excipient.
20. The pharmaceutical composition of embodiment 17, wherein said compound or salt thereof is butyric acid.
21. The pharmaceutical composition of embodiment 17, wherein said compound or salt thereof is 3-hydroxybutyric acid.
22. A compound of Formula (I): 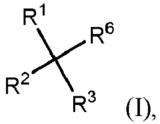 or a salt thereof, wherein:
   R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
   X is O, S, or N;
   Y is O, S, or N;
   R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
   R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
   R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
   A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
   R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S; and
   R⁸ is independently selected at each occurrence from hydrogen and C₁₋₂₀ alkyl.
23. The compound of embodiment 22, wherein said compound is represented by Formula (I-A): 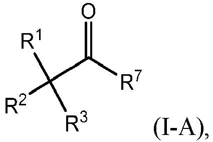 or a salt thereof.
24. A compound or a salt thereof listed in Table 1 or Table 2.
25. A method of combining a compound of Formula (I): 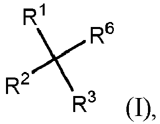 or a salt thereof, wherein:
   R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
   X is O, S, or N;
   Y is O, S, or N;
   R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
   R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
   R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
   A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
   R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S,
   R⁸ is independently selected at each occurrence from hydrogen and C₁₋₂₀ alkyl, and a pharmaceutically-acceptable excipient.
26. The method of embodiment 1, wherein said compound or a salt thereof is selected from: 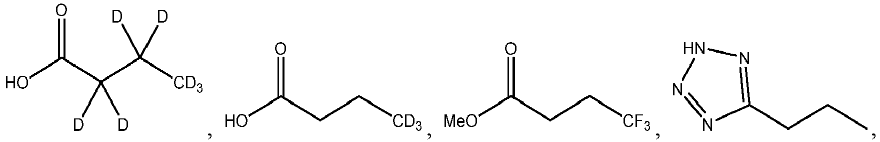 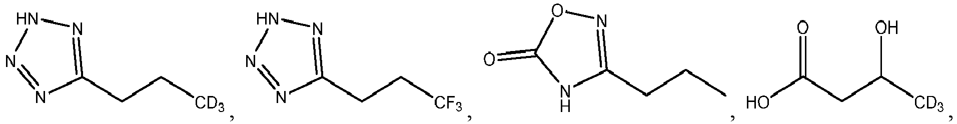 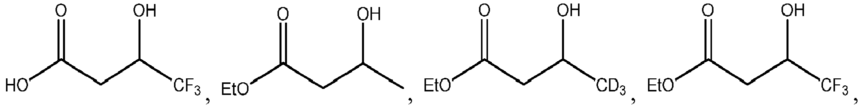 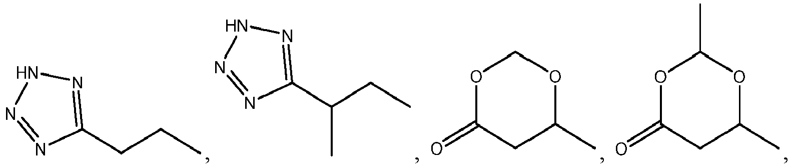 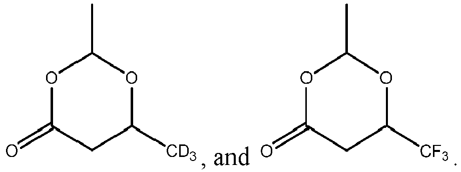
27. The method of embodiment 1, wherein said compound or a salt thereof is selected from:

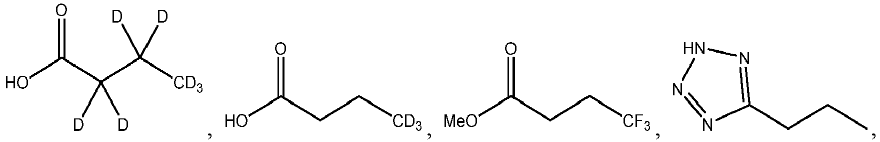

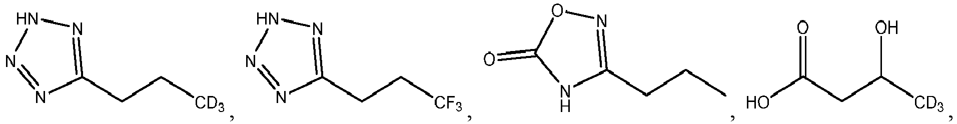

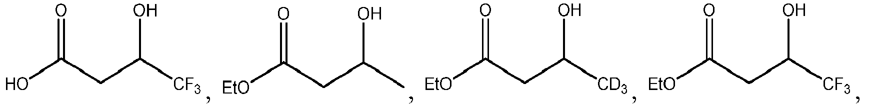

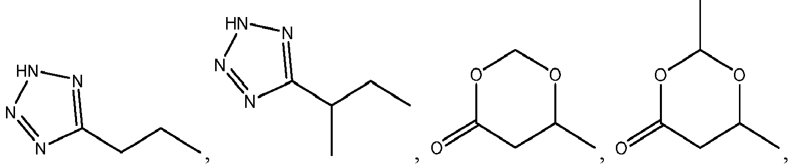

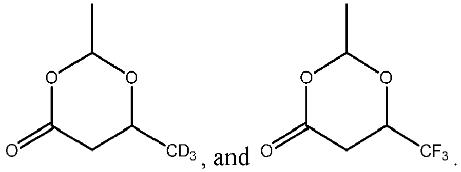

## Claims

1. A compound for use in a method of inducing sedation, sedating, treating a central nervous system disorder, treating a peripheral nervous system disorder, treating a convulsing disorder, treating a psychiatric disorder, treating ischemia, treating pain, treating spasticity, treating itching, or any combination thereof, the method comprising administering to a subject in need thereof a therapeutically-effective amount of a compound of Formula (I): 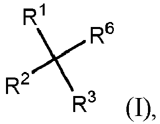 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S; and
R⁸ is independently selected at each occurrence from hydrogen and C₁₋₂₀ alkyl.

2. The compound for use of claim 1, wherein said compound is represented by Formula (I-A): 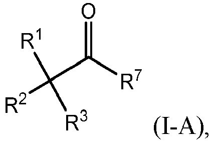 or a salt thereof.

3. The compound for use of claim 1, wherein the compound is a compound or a salt thereof listed in Table 1 or Table 2.

4. The compound for use of claim 1, wherein i) said compound or a salt thereof is a racemic mixture, ii) said compound or a salt thereof has an enantiomeric excess of greater than 80%, or iii) said compound or a salt thereof has a diastereomeric excess of greater than 80%.

5. The compound for use of claim 1, wherein said compound or a salt thereof has an alkene group, and wherein said alkene group has a cis geometry or a trans geometry.

6. The compound for use of claim 1, wherein said compound or a salt thereof is administered in a formulation.

7. The compound for use of claim 1, wherein said administering of a compound or a salt thereof alters distribution of lipids in a cell membrane.

8. The compound for use of claim 1, wherein said administering of a compound or a salt thereof alters cell membrane thickness.

9. The compound for use of claim 1, wherein said compound or salt thereof is selected from the group consisting of: (S)-3-hydroxybutanoic acid, (R)-3-hydroxybutanoic acid, 3,3-dimethylbutanoic acid, 2-benzamido-2-hydroxyacetic acid, butyric acid, 2-ethylmalonic acid, glutamine, and a salt of any one thereof.

10. The compound for use of claim 1, wherein said compound or salt thereof is selected from the group consisting of: butyric acid, 3-hydroxybutyric acid, ethylmalonic acid, diethyl ethyl malonate, ethyl (S)-(+)-mandelate, ethyl isovaleric acid, ethyl butyrate, diethyl ethylphenylmalonate, and a salt of any one thereof, preferably wherein said compound or salt thereof is selected from the group consisting of: butyric acid, 3-hydroxybutyric acid, ethylmalonic acid, diethyl ethyl malonate, and a salt of any one thereof, more preferably wherein said compound or salt thereof is butyric acid or 3-hydroxybutyric acid.

11. A pharmaceutical composition comprising a compound as defined in any one of claims 1 to 10 and a pharmaceutically-acceptable excipient.

12. A compound of Formula (I): 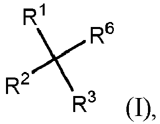 or a salt thereof, wherein:
R¹, R², R³ are independently selected at each occurrence from hydrogen, halogen, -X-R⁴, -N(R⁴)₂, -N(R⁴)C(X)R⁴, -C(X)R⁴, -C(X)YR⁴, -C(X)N(R⁴)₂, -CN, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, C₁₋₂₀ alkyl, C₂₋₂₀ alkenyl, C₂₋₂₀ alkynyl, -X-R⁴, - N(R⁴)₂, -C(X)R⁴, -C(X)YR⁴, -C(X)N(R⁴)₂, =O, =S, -CN, C₃₋₁₀ carbocycle, C₅₋₁₀ aryl, 3- to 10-membered heterocycle, and 3- to 10-membered heteroaryl; or R¹ and R² together form a C₃₋₁₀ carbocycle, C₅₋₁₀ aryl, 3- to 10-membered heterocycle, 3- to 10-membered heteroaryl, an oxo, or thio;
X is O, S, or N;
Y is O, S, or N;
R⁴ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁵, C₁₋₂₀ alkyl, C₃₋₁₀ carbocycle, C₅₋₁₀ aryl, 3- to 10-membered heterocycle, 3- to 10-membered heteroaryl, =O, and =S;
R⁵ is independently selected at each occurrence from hydrogen, C₁₋₂₀ alkyl, C₂₋₂₀ alkenyl, C₂₋₂₀ alkynyl, C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁷, C₁₋₂₀ alkyl, C₃₋₁₀ carbocycle, C₅₋₁₀ aryl, 3- to 10-membered heterocycle, 3- to 10-membered heteroaryl, =O, and =S;
R⁶ is independently selected at each occurrence from -A-R⁷, C₃₋₁₀ carbocycle, C₅₋₁₀ aryl, 3- to 10-membered heterocycle, and 3- to 10-membered heteroaryl, wherein each cycle in R⁶ is independently optionally substituted at each occurrence with one or more substituents selected from halogen, -X-R⁴, -N(R⁴)₂, -C(X)R⁴, -C(X)YR⁴, -C(X)N(R⁴)₂, -CN;
A is independently selected at each occurrence from -C(X)-, -C(X)NR⁵SO₂-, -P(O)(OR⁵)-, -SO₂-, -NR⁵-, -NR⁵C(X)-, -NR⁵C(X)NR⁵SO₂-, -NR⁵SO₂-, C₃₋₁₀ carbocycle, C₅₋₁₀ aryl, 3- to 10-membered heterocycle, and 3- to 10-membered heteroaryl, wherein each cycle in A is independently optionally substituted with one or more substituents selected from halogen, -X-R⁴, -N(R⁴)₂, -C(X)R⁴, -C(X)YR⁴, -C(X)N(R⁴)₂, -CN, =O, and =S;
R⁷ is independently selected at each occurrence from hydrogen, -OR⁸, -SR⁸, NHR⁸, and C₃₋₁₀ carbocycle, C₅₋₁₀ aryl, 3- to 10-membered heterocycle, and 3- to 10-membered heteroaryl, each of which is independently optionally substituted at each occurrence with one or more substituents selected from halogen, -OR⁸, C₁₋₂₀ alkyl, C₃₋₁₀ carbocycle, C₅₋₁₀ aryl, 3- to 10-membered heterocycle, 3- to 10-membered heteroaryl, =O, and =S; and
R⁸ is independently selected at each occurrence from hydrogen and C₁₋₂₀ alkyl.

13. The compound of claim 12, wherein said compound is represented by Formula (I-A): 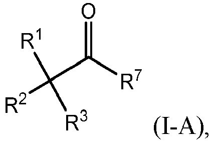 or a salt thereof.

14. The compound of claim 12, wherein said compound is a compound or a salt thereof listed in Table 1 or Table 2.

15. The compound for use of claim 1, wherein said compound or a salt thereof is selected from: 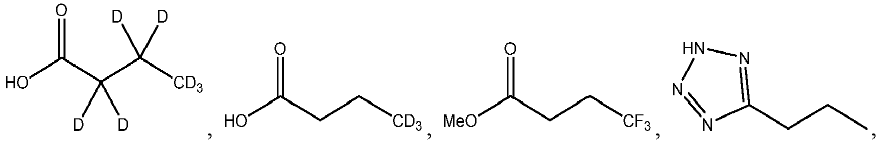 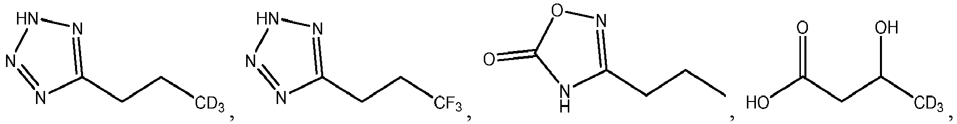 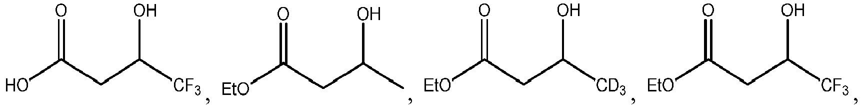 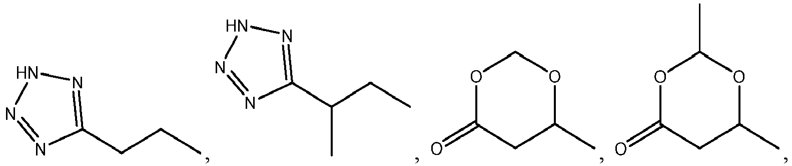 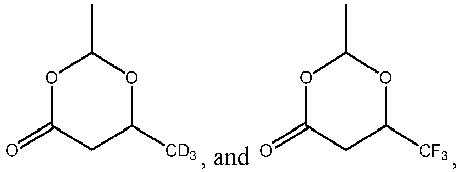 preferably wherein said compound or a salt thereof is selected from:
